# EUROPÄISCHE PATENTSCHRIFT

(11) **EP 3 448 973 B1**
(45) Veröffentlichungstag und Bekanntmachung des Hinweises auf die Patenterteilung: **04.03.2020**
(21) Anmeldenummer: 17721079.6
(22) Anmeldetag: 24.04.2017
(51) Int. Cl.: C11D 1/62, A61Q 5/12, A61K 8/898, C11D 3/00, C11D 3/37

(54) **ZUSAMMENSETZUNGEN ENTHALTEND CARBAMATO-FUNKTIONALISIERTE ORGANOPOLYSILOXANE UND KATIONISCHE TENSIDE**
COMPOSITIONS CONTAINING CARBAMATE FUNCTIONALIZED ORGANOPOLYSILOXANES AND CATIONIC SURFACTANTS
COMPOSITIONS CONTENANT DES ORGANOPOLYSILOXANES FONCTIONNALISÉS PAR CARBAMATO ET DES TENSIOACTIFS CATIONIQUES

(30) Priorität: 26.04.2016 DE 102016207063
(43) Veröffentlichungstag der Anmeldung: 06.03.2019
(73) Patentinhaber: Wacker Chemie AG, 81737 München (DE)
(72) Erfinder: BREHM, Christof, 84489 Burghausen (DE); BECKER, Richard, Ann Arbor, MI 48103 (US); HÖLZLWIMMER, Elisabeth, 84359 Simbach (DE); STALLEICHER, Christine, 84489 Burghausen (DE)
(74) Vertreter: Deffner-Lehner, Maria
(86) Internationale Anmeldenummer: PCT/EP2017/059652
(87) Internationale Veröffentlichungsnummer: WO 2017/186638

(56) Entgegenhaltungen:
- EP-A1- 0 563 961
- EP-A1- 0 639 369
- EP-A2- 0 459 821
- WO-A1-2009/150213

## Beschreibung

Die Erfindung betrifft Zusammensetzungen enthaltend Carbamato-funktionalisierte Organopolysiloxane und deren Verwendung für die Behandlung von Fasern, insbesondere von Textilfasern und Textilgeweben.

Viele Formulierungen werden in "Consumer Products" eingesetzt, um einen bestimmten Nutzen zu erzielen. Beispiele sind eine besondere Weichheit, eine verbesserte Haptik oder die Reduktion von Falten.

Typischerweise enthalten derartige Formulierungen wasserunlösliche, quartäre Ammoniumverbindungen mit in der Regel mindestens zwei langkettigen Alkyl- oder Alkenylketten. Aufgrund ihrer besseren biologischen Abbaubarkeit haben solche quartäre Ammoniumverbindungen an Interesse gewonnen, die langkettige Alkyl- oder Alkenyl-Gruppen enthalten, die durch funktionelle Gruppen, wie beispielsweise Carboxygruppen, unterbrochen sind. Derartige Verbindungen sind seit langem bekannt und werden beispielsweise in DE 16 19 058 A1, DE 19 35 499 A1, US 3,915,867 oder EP 239 910 A2 beschrieben.

Auch sind Formulierungen mit Kombinationen aus Emulgatoren, auch kationischen Emulgatoren, und funktionalisierten Polydiorganosiloxanen, beispielsweise Amino-funktionalisierten Polydiorganosiloxanen, Polydiorganosiloxanen mit quartären Funktionalisierungen oder Hydroxypropylamino-funktionalisierten Polydiorganosiloxanen bekannt. Derartige Formulierungen sind beispielsweise in WO 2011/123727 A2 oder WO 2011/123737 A1 beschrieben.

Silicone als Bestandteile von Formulierungen in "Consumer Products" sind ebenfalls seit langem bekannt. Siliconenthaltende Formulierungen, welche Carbamato-funktionalisierte Polydiorganosiloxane enthalten, werden für die Textil-Ausrüstung beispielsweise in JP 2047371 A2 beschrieben. Die Weichheit wird bei den Textilien durch die Behandlung von nicht-ionisch stabilisierten Emulsionen von Carbamato-funktionalisierten Polydiorganosiloxane hervorgerufen. Es handelt sich bei den Carbamato-funktionalisierten Polydiorganosiloxanen um Reaktionsprodukte aus der Reaktion aus Amino-modifizierten Polydimethylsiloxanen der Aminzahl > 0,5 mmol/g und Mischungen aus Ethylencarbonat und Propylencarbonat, wobei mehr als 50 Mol% der Aminogruppen mit Carbamato-Gruppen funktionalisiert wurden. Eine derartig hohe Funktionalisierung von mehr als 50 Mol% der Aminogruppen ist notwendig, um eine Vergilbung des Textils zu verringern.

In WO 2009/150213 A1 wird die Verwendung von Carbamato-funktionalisierten Polydiorganosiloxanen für das Finishing organischer Fasern und Textilien beschrieben. Die in WO 2009/150213 A1 beschriebenen Carbamato-funktionalisierten Polydiorganosiloxane sind Reaktionsprodukte aus Amino-modifizierten Polydimethylsiloxanen der Aminzahl > 0,5 mmol/g mit Glycerincarbonat, bei denen sämtliche Aminogruppen funktionalisiert wurden.

Aus JP 2009-052154, JP 2009-052155 und JP 2010-202984 sind ebenfalls Formulierungen zum Textil-Finishing bekannt, wobei Carbamato-funktionalisierte Polydiorganosiloxane in Kombination mit ausgewählten nicht-ionischen Tensiden verwendet werden. Auch hier beruhen die Carbamato-funktionalisierten Polydiorganosiloxane auf Amino-modifizierten Polydimethylsiloxanen der Aminzahl > 0,5 mmol/g.

Es bestand die Aufgabe Zusammensetzungen, die Carbamato-funktionalisierte Polydiorganosiloxane enthalten und die zur Behandlung, insbesondere zur Pflege und Reinigung, von Fasern, wie Textilien, geeignet sind, bereitzustellen, wobei die Fasern nach deren Behandlung verbesserte Eigenschaften aufweisen, wie weicher Griff, schnelle Wasseraufnahme, geringe Knitterneigung und leichtes Bügeln, ohne dass eine Vergilbung beobachtet wird. Die Aufgabe wird durch die Erfindung gelöst.

Gegenstand der Erfindung sind Zusammensetzungen, enthaltend
(A) mindestens 0,1 Gew.-% und höchstens 10,0 Gew.-% Carbamato-funktionalisierte Organopolysiloxane, die durchschnittlich je Molekül mindestens eine Carbamato-funktionelle Gruppe Y der Formel

   -R⁴-[NX-R⁵-]ₙNX-H

   enthalten, wobei
   X gleich oder verschieden ist und ein Wasserstoffatom oder einen Rest Z der Formel

      -CO-O-CHR⁶-CH₂-OH

      oder

      -CO-O-CH₂-CHR⁶-OH

      bedeutet, wobei durchschnittlich je Molekül mindestens ein Rest X ein Rest Z ist,
   wobei R⁴ gleich oder verschieden ist und einen zweiwertigen, Si-C-gebundenen Kohlenwasserstoffrest mit 1 bis 18 C-Atomen bedeutet,
   R⁵ gleich oder unterschiedlich ist und einen zweiwertigen Kohlenwasserstoffrest mit 1 bis 6 C-Atomen bedeutet,
   R⁶ gleich oder unterschiedlich ist und ein Wasserstoffatom oder einen einwertigen Kohlenwasserstoffrest mit 1 bis 36 C-Atomen bedeutet, wobei eine oder mehrere -CH₂-Gruppen durch Heteroatome, vorzugsweise durch -O- oder -S-, ersetzt sein können,
   n gleich 1, 2, 3 oder 4, vorzugsweise 1, ist,
(B) mindestens 1 Gew.-% und höchstens 80 Gew.-% kationische Tenside
   und
(C) mindestens 30 Gew.-% und höchstens 97 Gew.-% Wasser jeweils bezogen auf das Gesamtgewicht der wässrigen Zusammensetzungen.

Die erfindungsgemäßen Zusammensetzungen enthalten vorzugsweise mindestens 0,25 Gew.-%, bevorzugt mindestens 0,4 Gew.-%, und vorzugsweise höchstens 5 Gew.-%, bevorzugt höchstens 3 Gew.-%, Carbamato-funktionalisierte Organopolysiloxane (A).

Die erfindungsgemäßen Zusammensetzungen enthalten vorzugsweise mindestens 1,5 Gew.-%, bevorzugt mindestens 2,5 Gew.-%, und vorzugsweise höchstens 40 Gew.-%, bevorzugt höchstens 25 Gew.-%, kationische Tenside (B).

Die erfindungsgemäßen Zusammensetzungen enthalten vorzugsweise mindestens 45 Gew.-%, bevorzugt mindestens 60 Gew.-%, und vorzugsweise höchstens 97 Gew.-%, bevorzugt höchstens 95 Gew.-%, Wasser (C).

Vorzugsweise werden die Carbamato-funktionalisierten Organopolysiloxane (A) in Form ihrer wässrigen Emulsionen eingesetzt. Die Stabilisierung der wässrigen Emulsionen der Carbamato-funktionalisierten Organopolysiloxane (A) erfolgt unter Verwendung von nicht-ionischen Emulgatoren und/oder kationischen Emulgatoren und/oder Schutzkolloiden. Insbesondere die Verwendung von nicht-ionischen Emulgatoren oder einer Kombination aus nicht-ionischen und kationischen Emulgatoren zur Stabilisierung der Emulsionen ist bevorzugt, da sie eine besonders hohe Verträglichkeit in der erfindungsgemäßen Zusammensetzung erzeugen.

Carbamato-funktionalisierte Polydiorganosiloxane (A) sind seit langem bekannt und beispielsweise in JP 2047371 A2 beschrieben.

In den erfindungsgemäßen Zusammensetzungen werden vorzugsweise Carbamato-funktionalisierte Organopolysiloxane (A) eingesetzt, die folgende Struktureinheiten enthalten:
M [R¹₂R²SiO_{1/2}] und/oder M' [R¹₂(Y)SiO_{1/2}]
   und
D [R¹₂SiO_{2/2}] und/oder D' [R²(Y)SiO_{2/2}] und/oder T' [(Y)SiO_{3/2}] und gegebenenfalls
T [R¹SiO_{3/2}] und/oder
Q [SiO_{4/2}]
mit der Maßgabe, dass durchschnittlich je Molekül mindestens eine Struktureinheit mit einer Carbamato-funktionellen Gruppe Y enthalten ist und wobei durchschnittlich je Molekül mindestens eine Gruppe Y einen Rest Z enthält,
   wobei
R¹ gleich oder verschieden ist und einen einwertigen Si-C-gebundenen Kohlenwasserstoffrest mit 1 bis 18 C-Atomen bedeutet,
R² gleich oder unterschiedlich ist und gleich Rest R¹ oder eine Hydroxygruppe -OH oder eine Alkoxy-Gruppe der Formel -O-R³ bedeutet, wobei R³ ein gegebenenfalls substituierter Alkylrest mit 1-8 C-Atomen ist, und
Y die oben dafür angegebene Bedeutung hat.

In den erfindungsgemäßen Zusammensetzungen werden bevorzugt Carbamato-funktionalisierte Polydiorganosiloxane (A) der folgenden Formel eingesetzt

[R¹₂R²SiO_{1/2}]₂[R²(Y)SiO_{2/2}]ₖ[R¹₂SiO_{2/2}]ₘ (I),

wobei
R¹, R² und Y die oben dafür angegebene Bedeutung haben,
wobei durchschnittlich je Molekül mindestens eine Gruppe Y einen Rest Z enthält,
m eine ganze Zahl und mindestens 40, vorzugsweise mindestens 65, bevorzugt mindestens 110, und höchstens 1000, vorzugsweise höchstens 800, bevorzugt höchstens 500, ist
k eine ganze Zahl und mindestens 1, vorzugsweise mindestens 2, und höchstens 40, vorzugsweise höchstens 20, bevorzugt höchstens 10, ist,
wobei das Verhältnis von m zu k mindestens 25, vorzugsweise mindestens 40, bevorzugt mindestens 70, und höchstens 1000, vorzugsweise höchstens 500, bevorzugt höchstens 150 ist.

Bei den Carbamato-funktionalisierten Organopolysiloxanen (A), insbesondere Carbamato-funktionalisierten Polydiorganosiloxanen (A), sind vorzugsweise mindestens 5 Mol%, bevorzugt mindestens 15 Mol%, besonders bevorzugt mindestens 20 Mol%, und vorzugsweise weniger als 50 Mol%, bevorzugt weniger als 40 Mol%, besonders bevorzugt weniger als 30 Mol% der N-gebundenen Reste X in den Gruppen Y nicht ein Wasserstoffatom, sondern haben die Bedeutung des Restes Z.

Dies bedeutet, dass vorzugsweise mindestens 5 Mol% und vorzugsweise weniger als 50 Mol% der Aminogruppen mit Resten Z, also Carbamatogruppen, funktionalisiert sind.

Gegebenenfalls können in den Carbamato-funktionalisierten Polydiorganosiloxanen (A) der Formel (I) auch geringe Mengen an Struktureinheiten T oder Q enthalten sein.

Die in den erfindungsgemäßen Zusammensetzungen eingesetzten Carbamato-funktionalisierten Organopolysiloxane (A) werden vorzugsweise durch Umsetzung von Amino-funktionalisierten Organopolysiloxanen (A'), die durchschnittlich je Molekül mindestens eine Gruppe Y' der Formel

-R⁴-[NH-R⁵-]ₙNH₂

enthalten, wobei R⁴, R⁵ und n die oben dafür angegebene Bedeutung haben,
mit cyclischen Carbonaten hergestellt.

Zur Herstellung der Carbamato-funktionalisierten Organopolysiloxane (A) werden bevorzugt Amino-funktionalisierte Polydiorganosiloxane (A') der folgenden Formel eingesetzt

[R¹₂R²SiO_{1/2}]₂[R²(Y')SiO_{2/2}]ₖ[R¹₂SiO_{2/2}]ₘ (I'),

wobei
R¹, R² , m, und k die oben angegebene Bedeutung aufweisen, Y' eine Gruppe der allgemeinen Formel

-R⁴-[NH-R⁵-]ₙNH₂

ist, wobei R⁴, R⁵ und n die oben dafür angegebene Bedeutung haben.

Als cyclische Carbonate werden vorzugsweise solche der folgenden Formel eingesetzt 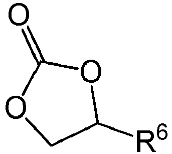 wobei,
R⁶ die oben dafür angegebene Bedeutung hat.

Zur Herstellung der Carbamato-funktionalisierten Organopolysiloxane (A) wird die Menge an eingesetztem cyclischem Carbonat so ausgewählt, dass im hergestellten Carbamato-funktionalisierten Organopolysiloxan (A) vorzugsweise mindestens 5 Mol%, bevorzugt mindestens 15 Mol%, besonders bevorzugt mindestens 20 Mol%, und vorzugsweise weniger als 50 Mol%, bevorzugt weniger als 40 Mol%, besonders bevorzugt weniger als 30 Mol% der N-gebundenen Reste X in den Gruppen Y zu Resten Z umgesetzt werden.

Beispiele für Kohlenwasserstoffreste R¹ sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl- und Methylcyclohexylreste.

Bevorzugte Beispiele für R¹ sind der Methyl-, Ethyl-, Octyl- und Dodecylrest. Ein besonders bevorzugtes Beispiel für R¹ ist der Methylrest.

Beispiele für Kohlenwasserstoffreste R² sind die Reste wie für Rest R¹ beschrieben oder eine Hydroxygruppe -OH oder eine Alkoxy-Gruppe der Formel -O-R³ mit R³ gleich einem Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest.
Bevorzugte Beispiele für R² sind der Methylrest, der Ethylrest, die Hydroxygruppe, der Methoxyrest und der Ethoxyrest.

Beispiele für R⁴ sind zweiwertige Kohlenwasserstoffreste wie die Methylengruppe, die 1,2-Ethylengruppe, die 1,3-Propylengruppe, die 1,3-Butylengruppe, die 1,4-Butylengruppe, die 1,5-Pentylengruppe, die 1,6-Hexylengruppe.
Besonders bevorzugte Beispiele sind die 1,3-Propylengruppe und die 1,3-Butylengruppe.

Beispiele für R⁵ sind zweiwertige Kohlenwasserstoffreste wie die 1,2-Ethylengruppe, die 1,3-Propylengruppe, die 1,3-Butylengruppe, die 1,4-Butylengruppe, die 1,5-Pentylengruppe, die 1,6-Hexylengruppe.

Ein besonders bevorzugtes Beispiel ist die 1,2-Ethylengruppe.

R⁶ ist vorzugsweise ein Wasserstoffatom oder ein einwertiger, gegebenenfalls durch -O- substituierter, Kohlenwasserstoffrest mit 1 bis 10 C-Atomen, bevorzugt mit 1 bis 6 C-Atomen, besonders bevorzugt ein Alkyl- oder Alkoxyalkylrest mit 1 bis 10 C-Atomen, insbesondere mit 1 bis 6 C-Atomen. Bevorzugtes Beispiel für R⁶ ist der Methylrest.

Bevorzugte Beispiele für die Reste Y' sind

- (CH₂)₂NH(CH₂)₂NH₂,

- (CH₂)₃NH(CH₂)₂NH₂,

- (CH₂)₃NH(CH₂)₃NH₂,

- (CH₂-CH(CH₃)-CH₂-)NH(CH)₂NH₂

und

- (CH₂)₄NH(CH₂)₄NH₂ .

Besonders bevorzugte Beispiele für die Reste Y' sind

- (CH₂)₃NH(CH₂)₂NH₂,

und

- (CH₂-CH(CH₃)-CH₂-)NH(CH)₂NH₂.

Die Indices k und m in Formel (I') werden so gewählt, dass die Viskosität der eingesetzten Amino-funktionalisierten Polydiorganosiloxane (A') bei vorzugsweise mindestens 50 mPas, bevorzugt mindestens 100 mPas, besonders bevorzugt mindestens 250 mPas, jeweils gemessen bei 25°C und einem Schergefälle von 10/s, und vorzugsweise höchstens 100000 mPas, bevorzugt höchstens 50000 mPas, besonders bevorzugt höchstens 10000 mPas, jeweils gemessen bei 25°C und einem Schergefälle von 5/s, liegt.

Das Verhältnis von k und m wird so gewählt, dass die eingesetzten Amino-funktionalisierten Polydiorganosiloxane (A') eine Aminzahl von vorzugsweise mindestens 0,1 mmol/g, bevorzugt mindestens 0,15 mmol/g, besonders bevorzugt mindestens 0,2 mmol/g, und vorzugsweise höchstens 1,0 mmol/g, bevorzugt höchstens 0,7 mmol/g, besonders bevorzugt höchstens 0,4 mmol/g aufweisen.

Die zur Herstellung der Carbamato-funktionalisierten Polydiorganosiloxane (A) eingesetzten cyclischen Carbonate sind entweder kommerziell erhältlich oder können synthetisiert werden, wie beispielsweise in US 3642858 A beschrieben.

Typischerweise werden als cyclische Carbonate 1,2-Alkylencarbonate oder Alkoxyalkyl-substituierte Ethylencarbonate eingesetzt.
Bevorzugte cyclische Carbonate sind solche Carbonate mit
R⁶ gleich einem Wasserstoffatom oder einem einwertigen, gegebenenfalls durch -O- substituierten, Kohlenwasserstoffrest mit 1 bis 10 C-Atomen, besonders bevorzugt mit 1 bis 6 C-Atomen.
Beispiele sind Ethylencarbonat, 1,2-Propylencarbonat, 1,2-Butylencarbonat, 1,2-Pentylencarbonat, 1,2-Hexylencarbonat, 1,2-Octylencarbonat, 1,2-Dodecylencarbonat, 3-Methyl-1,2-butylencarbonat, 3-Methyl-1,2-Pentylencarbonat, 3-Ethyl-1,2-Pentylencarbonat, 4-Methyl-1,2-Pentylencarbonat, 5-Methyl-1,2-Hexylencarbonat, 3-Methoxy-1,2-Propylencarbonat, 3-Ethoxy-1,2-Propylencarbonat, 3-n-Propoxy-1,2-propylencarbonat, 4-Methoxy-1,2-butylencarbonat, 4-Ethoxy-1,2-butylencarbonat, S-Methoxy-3-butylencarbonat, 5-Methoxy-1,2-Heptylencarbonat.

Bevorzugte cyclische Carbonate sind Ethylencarbonat, 1,2-Propylencarbonat, 3-Methoxy-1,2-Propylencarbonat.
Ein besonders bevorzugtes cyclisches Carbonat ist das 1,2-Propylencarbonat.

Bei den in den erfindungsgemäßen Zusammensetzungen verwendeten kationischen Tensiden handelt es sich vorzugsweise um
(B1) Quartäre Ammonium-Tenside der Formel 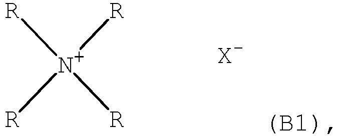
   wobei R unabhängig voneinander ein Wasserstoffatom, eine Alkylgruppe, eine Alkenylgruppe, eine Hydroxyalkylgruppe, eine Benzylgruppe oder eine Gruppe der Formel -(CₚH₂ₚO)_{q}-H ist, wobei p eine ganze Zahl von 1 bis 4 und q eine ganze Zahl von 2 bis 5 ist, und
   X⁻ ein Halogenid-, ein Sulfat-, ein Sulfonat-, ein Nitrat-, ein Methylsulfat- oder ein Ethylsulfat-Anion, vorzugsweise ein Chlorid- oder Methylsulfat-Anion ist,
   oder
(B2) Ester-/Amido-haltige quartäre Ammonium-Tenside der Formel 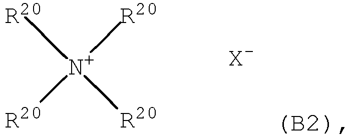
   wobei mindestens ein Rest R²⁰ ein einwertiger Kohlenwasserstoffrest ist, der eine Gruppe G der Formel - O-(O)C-, -C(O)-O-, -NR-C(O)-, oder -C(O)-NR- oder -CR=NR enthält,
   und die weiteren Resten R²⁰ unabhängig voneinander eine Gruppe R sind
   oder ein Rest der Formel -R^{20'}-N⁺R²⁰₃ X⁻ sind,
   wobei R^{20'} eine Alkylen-Gruppe ist, die zwei Ammonium-Gruppen R²⁰₃N⁺ X⁻ miteinander zu einer Verbindung R²₃N-Alkylen-NR²₃ 2X⁻ verknüpft,
   oder zwei Reste R²⁰ einen gegebenenfalls ungesättigten Ring formen, und
   R und X⁻ die oben dafür angegebene Bedeutung haben.

Ester-/Amido- oder Imido-haltige quartäre Ammonium-Tenside der Formel (B2) sind bevorzugt.

Bei den in den erfindungsgemäßen Zusammensetzungen verwendeten kationischen Tensiden kann es sich um eine Art von Tensid aber auch um mehrere Arten von Tensiden handeln.

Bei den quartären Ammonium-Tensiden (B1) handelt es sich um
(B1-1) Tenside der Formel

   R²¹R²²R²³R²⁴N⁺ X⁻ (B1-1),

   wobei
   R²¹, R²² unabhängig voneinander eine Alkylgruppe mit 1 bis 4 C-Atomen, eine Hydroxyalkylgruppe mit 1 bis 4 C-Atomen, eine Benzylgruppe oder eine Gruppe der Formel -(CₚH₂ₚO)_{q}-H bedeutet,
   wobei p eine ganze Zahl von 1 bis 4 und q eine ganze Zahl von 2 bis 5 ist,
   R²³, R²⁴ unabhängig voneinander eine Alkyl- oder Alkenylgruppe mit 8 bis 22 C-Atomen oder
   R²³ eine Alkylgruppe mit 8 bis 22 C-Atomen und
   R²⁴ eine Alkylgruppe mit 1 bis 10 C-Atomen, eine Hydroxyalkylgruppe mit 1 bis 10 C-Atomen, eine Benzylgruppe oder eine Gruppe der Formel -(CₚH₂ₚO)_{q}-H bedeutet, wobei p und q die oben dafür angegebene Bedeutung haben, und
   X⁻ ein Halogenid, ein Sulfat-, ein Sulfonat-, ein Nitrat-, ein Methylsulfat- oder ein Ethylsulfat-Anion, insbesondere ein Chlorid- oder Methylsulfat-Anion ist,
   oder um
(B1-2) Tenside der Formel

   R²⁵₍₄₋ᵣ₎R²⁶ᵣN⁺ X⁻ (B1-2),

   wobei
   R²⁵ unabhängig voneinander ein Wasserstoffatom, eine Alkyl-gruppe mit 1 bis 3 C-Atomen, beispielsweise die Methyl-, die Ethyl-, die Propylgruppe, eine Hydroxyalkylgruppe mit 1 bis 3 C-Atomen, beispielsweise die Hydroxyethylgruppe, eine Benzylgruppe oder eine Gruppe der Formel -(CₚH₂ₚO)_{q}-H bedeutet, wobei p eine ganze Zahl von 1 bis 4, bevorzugt 2 ist, und q eine ganze Zahl von 2 bis 5 ist,
   R²⁶ ein Kohlenwasserstoffrest mit 12 bis 24 C-Atomen, insbesondere ein Alkyl- oder Alkenylrest mit 12 bis 24 C-Atomen ist,
   r 2 oder 3 ist, und
   X⁻ die oben dafür angegebene Bedeutung hat.

Bei den Ester-/Amido-haltigen quartären Ammonium-Tensiden (B2) handelt es sich vorzugsweise um solche ausgewählt aus der Gruppe von
(B2-1) Tenside der Formel

   R²⁵₍₄₋ᵣ₎(R²⁶-G¹-R²⁷)ᵣN⁺ X⁻ (B2-1),

   wobei
   R²⁵ unabhängig voneinander ein Wasserstoffatom, eine Alkylgruppe mit 1 bis 3 C-Atomen, beispielsweise die Methyl-, die Ethyl-, die Propylgruppe, eine Hydroxyalkylgruppe mit 1 bis 3 C-Atomen, beispielsweise die Hydroxyethylgruppe, eine Benzylgruppe oder eine Gruppe der Formel -(CₚH₂ₚO)_{q}-H bedeutet, wobei p eine ganze Zahl von 1 bis 4, bevorzugt 2 ist, und q eine ganze Zahl von 2 bis 5 ist,
   R²⁶ ein einwertiger Kohlenwasserstoffrest, bevorzugt ein Alkyl- oder Alkenylrest mit 12 bis 24 C-Atomen, ist,
   R²⁷ ein zweiwertiger, linearer oder verzweigter Alkylenrest mit 1 bis 6 C-Atomen, bevorzugt mit 1 bis 3 C-Atomen, insbesondere die Ethylen-, die n-Propylen- oder die i-Propylen-Gruppe, ist,
   G¹ eine Gruppe -O-(O)C-, -C(O)-O-, -NR-C(O)-, oder -C(O)NR-ist,
   r 2 oder 3 ist und
   X⁻ die oben dafür angegebene Bedeutung hat,
(B2-2) Tenside der Formel 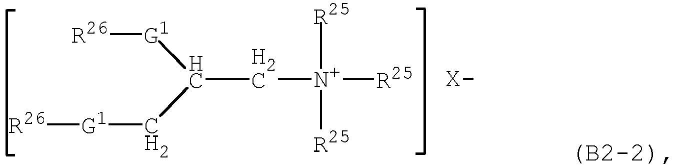 wobei
   R²⁵, R²⁶, G¹ und X⁻ die oben dafür angegebene Bedeutung haben,
   insbesondere um Tenside der Formel 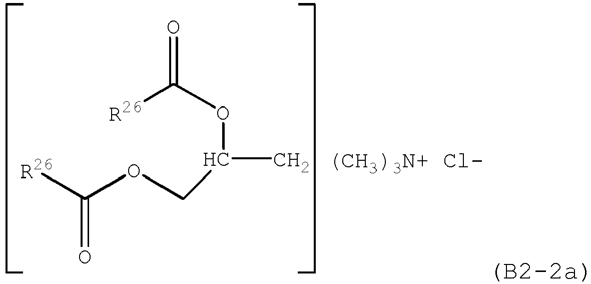 wobei
   R²⁶ die oben dafür angegebene Bedeutung hat,
   wobei im Falle, dass das Tensid (B2-2a) als Diester beschrieben ist, auch Monoester enthalten sein können, (wobei derartige Tenside und allgemeine Methoden zu deren Herstellung in US 4, 137, 180 beschrieben sind),
(B2-3) Tenside der Formel 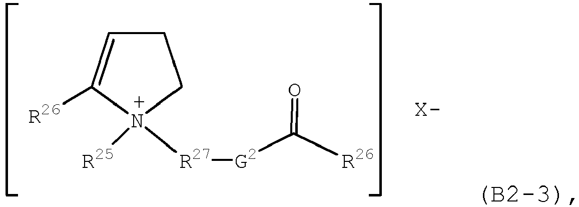 wobei
   G² ein Sauerstoff-Atom oder die Gruppe NR²⁵ ist,
   R²⁵, R²⁶, R²⁷ und X⁻ die oben dafür angegebene Bedeutung haben,
(B2-4) Tenside der Formel 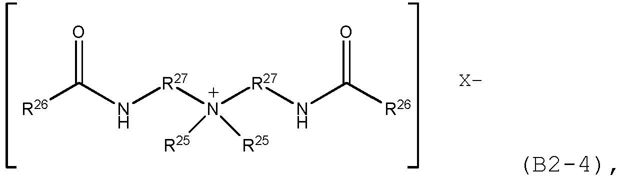 wobei
   R²⁵, R²⁶, R²⁷ und X⁻ die oben dafür angegebene Bedeutung haben,
   (wobei derartige quartäre Tenside in US 5,296,622 beschrieben sind),
(B2-5) Tenside der Formel 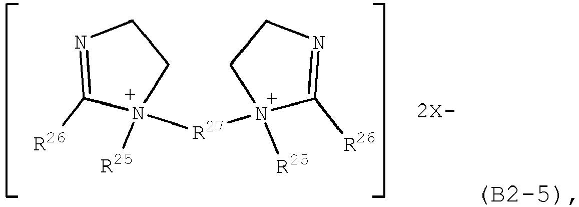 wobei
   R²⁵, R²⁶, R²⁷ und X⁻ die oben dafür angegebene Bedeutung haben,
   und deren Mischungen.

Beispiele für Tenside der Formel (B1-1) sind
(a) Monoalkyl-quartäre Ammoniumsalze, wie z.B.
   - das Behenyltrimethylammonium-Salz,
   - das Stearyltrimethylammonium-Salz
   - das Cetyltrimethylammonium-Salz und
   - das hydrierte Tallölalkyltrimethylammonium-Salz
   und
(b) Dialkyl-quartäre Ammoniumsalze, wie z.B.
   - Dialkyl (C₁₄-C₁₈) dimethylammoniumchlorid,
   - Ditalgalkyl-dimethylammoniumchlorid,
   - Distearyldimethylammoniumchlorid und
   - Dicetyldimethylammoniumchlorid.

Beispiele für Tenside der Formel (B1-2) sind Dialkyldimethylammoniumsalze, wie Dioleyldimethylammoniumchlorid (erhältlich von Witco Corporation unter dem Markenname Adogen(R) 472).

Beispiele für Tenside der Formel (B2-1) sind N,N-bis(Stearoyl-oxyethyl)-N,N-dimethyl ammoniumchlorid, N,N-bis(Talloyl-oxyethyl)-N,N-dimethyl ammoniumchlorid, N,N-bis(Stearoyl-oxyethyl)-N-(2-hydroxyethyl)-N-methyl ammoniummethylsulfat und N,N-bis[Ethyl(tallowate)]-N-(2-hydroxyethyl)-N-methylammoniummethylsulfat.

Tenside der Formel (B2-2) werden mit DEQ abgekürzt oder auch als "Propyl" Esterquat der allgemeinen Bezeichnung 2,3-Di(acyloxy)-propyl-trimethylammoniumchlorid bezeichnet. Ein Beispiel für Tenside der Formel (B2-2) ist 2,3-Di(stearoyloxy)propyl-trimethylammoniumchlorid.

Ein Beispiel für Tenside der Formel (B2-3) ist 1-Methyl-l-stearoylamidoethyl-2-stearoylimidazolinium methylsulfat (erhältlich von der Witco Corporation unter dem Markennamen Varisoft(R)).

Beispiele für Tenside der Formel (B2-4) sind Difettsäureamidoamin-basierte Produkte der Formel:

[R²⁶-C(O)-NH-CH₂CH₂-N(CH₃)(CH₂CH₂OH)-CH₂CH₂-NH-C(O)-R²⁶]⁺ CH₃SO₄⁻

wobei R²⁶ die oben dafür angegebene Bedeutung hat,
z.B. ein Produkt erhältlich von der Fa. Witco Corporation unter dem Markennamen Varisoft(R) 222LT.

In den erfindungsgemäßen Zusammensetzungen können neben den Carbamato-funktionalisierten Polydiorganosiloxanen (A) und kationischen Tensiden (B) weitere Stoffe enthalten sein, wie Parfüms, Elektrolyte, nichtwässrige Lösungsmittel, Deponierungshilfsmittel, pH-Regulierungsmittel, Farbstoffe oder Schauminhibitoren.

Beispiele für weitere Stoffe sind Parfüms. Parfüms können in einer Menge von vorzugsweise 0,01 bis 3 Gew.-%, bevorzugt in Mengen von 0,1 bis 2,5 Gew.-% und besonders bevorzugt in Mengen von 0,2 bis 1,5 Gew.-%, jeweils bezogen auf das Gesamtgewicht der erfindungsgemäßen Zusammensetzungen, mitverwendet werden. Als Parfümöle bzw. Duftstoffe können einzelne Riechstoffverbindungen, z. B. die synthetischen Produkte vom Typ der Ester, Ether, Aldehyde, Ketone, Alkohole und Kohlenwasserstoffe verwendet werden. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind.

Beispiele für weitere Stoffe sind Elektrolyte. Elektrolyte können in einer Menge von vorzugsweise 0,01 bis 5 Gew.-% bezogen auf das Gesamtgewicht der erfindungsgemäßen Zusammensetzungen, mitverwendet werden. Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den erfindungsgemäßen Zusammensetzungen bevorzugt.

Beispiele für weitere Stoffe sind nichtwässrige Lösungsmittel. Nichtwässrige Lösungsmittel können in einer Menge von vorzugsweise 0,25 bis 15 Gew.-%, bevorzugt 0,25 bis 12 Gew.-%, besonders bevorzugt 0,25 bis 9 Gew.-%, jeweils bezogen auf das Gesamtgewicht der erfindungsgemäßen Zusammensetzungen, mitverwendet werden.

Die nichtwässrigen Lösungsmittel, die in den erfindungsgemäßen Zusammensetzungen eingesetzt werden können, stammen beispielsweise aus der Gruppe der ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Glycol, Propan- oder Butandiol, Glycerin, Diglycol, Propyl- oder Butyldiglycol, Hexylenglycol, Ethylenglycolmethylether, Ethylenglycolethylether, Ethylenglycolpropylether, Ethylenglycolmono-n-butylether, Diethylenglycolmethylether, Diethylenglycolethylether, Propylenglycolmethylether, Propylenglycolethylether, Propylenglycolpropylether, Methoxytriglycol, Ethoxytriglycol, Butoxytriglycol, Dipropylenglycolmonomethylether, Dipropylenglycolmonoethylether, 1-Butoxyethoxy-2-propanol; 3-Methyl-3-methoxybutanol, Propylen-glycol-t-butylether sowie Mischungen dieser Lösungsmittel.

Beispiele für weitere Stoffe sind Deponierungshilfsmittel. Deponierungshilfsmittel können in einer Menge von vorzugsweise 0,01 Gew.-% bis 10 Gew.-%, bevorzugt von 0,05 Gew.-% bis 5 Gew.-%, besonders bevorzugt von 0,15 Gew.-% bis 3 Gew.-%, jeweils bezogen auf das Gesamtgewicht der erfindungsgemäßen Zusammensetzungen, mitverwendet werden.

Geeignete Deponierungshilfsmittel sind beispielsweise in der Anmeldung US 12 080 358 beschrieben.
Bei den Deponierungshilfsmitteln kann es sich sowohl um kationische wie auch um amphotere Polymere handeln. Kationische Polymere und Verfahren zu ihrer Herstellung sind im Allgemeinen in der Literatur bekannt. Das kationische Polymer kann eine kationische Ladungsdichte von etwa 0,005 bis etwa 23 meq./g, bevorzugt von etwa 0,01 bis etwa 12 meq./g, besonders bevorzugt von etwa 0,1 bis etwa 7 meq./g bei gegebenem pH-Wert der Zusammensetzung aufweisen. Bei Amin-haltigen Polymeren hängt die Ladungsdichte vom pH-Wert der Zusammensetzungen ab, weshalb die Ladungsdichte beim beabsichtigten pH-Wert des Produktes gemessen wird. Entsprechende pH-Werte liegen im Allgemeinen in einem Bereich von etwa 2 bis etwa 11, insbesondere von etwa 2,5 bis etwa 9,5. Die Ladungsdichte wird berechnet, indem die Anzahl der Nettoladungen pro Wiederholungseinheit durch das Molekulargewicht der Wiederholungseinheit geteilt wird. Die positiven Ladungen können sich am Rückgrat der Polymere und/oder an den Seitenketten der Polymere befinden.

Beispiele für Deponierungshilfsmittel sind kationische oder amphotere Polysaccharide, Proteine und synthetische Polymere.

Kationische Polysaccharide können kationische Cellulosederivate, kationische Guar Gum-Derivate, Chitosan und dessen Derivate sowie kationische Stärken umfassen.
Kationische Polysaccharide haben ein Molekulargewicht von etwa 50.000 bis etwa 3.500.000. Zu geeigneten kationischen Polysacchariden gehören kationische Cellulose-Ether, insbesondere kationische Hydroxyethylcellulose und kationische Hydroxypropylcellulose. Beispiele für kationische Hydroxyalkylcellulose sind (Bezeichnung nach INCI) Polyquaternium 10 (z.B. erhältlich unter den Handelsnamen Ucare™ Polymer JR 30M, JR 400, JR 125, LR 400 and LK 400 der Fa. Amerchol Corp.), Polyquaternium 67 (z.B. erhältlich unter dem Handelsnamen Softcat SK™ der Fa. Amerchol Corp.) und Polyquaternium 4 (z.B. erhältlich unter den Handelsnamen Celquat™ H200 und Celquat L-200™ der Fa. National Starch and Chemical Company).
Weitere geeignete Polysaccharide sind mit Glycidyl-C₁₂-C₂₂-Alkyldimethylammoniumchlorid quaternisierte Hydroxyethyl-cellulose oder Hydoxypropylcellulose. Ein Beispiel hierzu ist (Bezeichnung nach INCI) Polyquaternium 24 (z.B. erhältlich unter dem Handelsnamen Quaternium LM 200 der Fa. Amerchol Corp.).
Kationische Guar Gum-Derivate umfassen kationisch derivatisierte Galactomannane oder kationisches Johannisbrotkernmehl. Ein Beispiel für einen kationischen Guar Gum ist das quaternäre Ammoniumderivat des Hydroxypropylguars, beispielsweise erhältlich unter dem Handelsnamen Jaguar(R) C13, Jaguar(R) Excel (Fa. Rhodia) oder N-Hance(R) (Fa. Aqualon). Kationische Stärken wurden beispielsweise von D. B. Solarek in "Modified Starches, Properties and Uses" (CRC Press, 1986) und in US 7,135,451 beschrieben.

Eine weitere Gruppe geeigneter kationischer Polymere umfasst solche, die durch Polymerisation ethylenisch ungesättigter Monomere mit einem geeigneten Initiator oder Katalysator hergestellt werden (beispielhaft beschrieben in US 6,642,200). Geeignete Polymere dieser Gruppe sind Polyethylenimine und seine Derivate oder synthetische Polymere, die durch Polymerisation eines oder mehrerer kationischer Monomere hergestellt werden.
Derartige Monomere können sein N,N-Dialkylaminoalkylacrylat, N,N-Dialkylaminoalkylmethacrylat, N,N-Dialkylaminoalkylacrylamid, N,N-Dialkylaminoalkylmethacrylamid, quaterniertes N,N-Dialkylaminoalkylacrylat, quaternisiertes N,N-Dialkylaminoalkylmethacrylat, quaterniertes N,N-Dialkylaminoalkylacrylamid, quaterniertes N,N-Dialkylaminoalkylmethacrylamid, Methacryloamidopropylpentamethyl-l,3-propylen-2-ol-ammoniumdichlorid, N,N,N,N',N',N",N"-Heptamethyl-N"-3-(l-oxo-2-methyl-2-propenyl)aminopropyl-9-oxo-8-azo-decan-l,4,10-triammoniumtrichlorid, Vinylamin und dessen Derivaten, Allylamin und dessen Derivate, Vinylimidazol, quaterniertes Vinylimidazol oder Diallyldialkylammoniumchlorid und Kombinationen davon, und gegebenenfalls ein zweites Monomer, ausgewählt aus der Gruppe, bestehend aus Acrylamid, N,N-Dialkylacrylamid, Methacrylamid, N,N-Dialkylmethacrylamid, C₁-C₁₂-Alkylacrylat, C₁-C₁₂-Hydroxyalkylacrylat, Polyalkylenglykolacrylat, C₁-C₁₂-Alkylmethacrylat, C₁-C₁₂ Hydroxyalkylmethacrylat, Polyalkylenglykol, Vinylacetat, Vinylalkohol, Vinylformamid, Vinylacetamid, Vinylalkylether, Vinylpyridin, Vinylpyrrolidon, Vinylimidazol, Vinylcaprolactam und Derivate, Acrylsäure, Methacrylsäure, Maleinsäure, Vinylsulfonsäure, Styrolsulfonsäure, Acrylamidopropylmethansulfonsäure (AMPS) und ihre Salze. Durch Verwendung verzweigender und vernetzender Monomere kann das Polymer gegebenenfalls verzweigt oder vernetzt sein. Verzweigende und vernetzende Monomere können sein: Ethylenglycoldiacrylat, Divinylbenzol und Butadien. Ferner kann die Zusammensetzung amphotere Deponierungshilfsmittel umfassen, solange das Polymer eine positive Nettoladung besitzt. Derartige Polymere haben eine kationische Ladungsdichte von etwa 0,05 bis etwa 18 meq./g.

Beispiele geeigneter kationischer Polymere dieser Gruppe sind Poly(acrylamid-co-diallyldimethylammoniumchlorid), Poly(acrylamido-methacrylamidopropyltrimethylammoniumchlorid), Poly(acrylamido-co-N,N-dimethylaminoethylacrylat) und seine quaternisierten Derivate, Poly(acrylamid-co-N,N-dimethylaminoethylmethacrylat) und dessen quaternisierte Derivate, Poly(hydroxyethylacrylat-co-dimethylaminoethylmethacrylat), Poly(hydroxypropylacrylate-co-dimethylaminoethylmethacrylat), Poly(co-hydroxypropylacrylatemethacrylamidopropyltrimethylammoniumchlorid), Poly(acrylamid-co-diallyldimethylammoniumchlorid-co-acrylsäure), Poly(acrylamid-methacrylamidopropyltrimethylammoniumchlorid-co-acrylsäure), Poly(diallyldimethylammoniumchlorid), Poly(vinylpyrrolidon-co-dimethylaminoethylmethacrylat), Poly(ethylmethacrylat-co-quaternisiertes dimethylaminoethylmethacrylat), Poly(ethylmethacrylat-co-oleyl-methacrylat-co-diethylaminoethylmethacrylat), Poly(diallyldimethylammoniumchlorid-co-acrylsäure), Poly(vinylpyrrolidon-co-quaterniertes vinylimidazol) und Poly(acrylamid-co-methacryloamidopropylpentamethyl-1,3-propylen-2-ol-ammoniumchlorid).
Geeignete Deponierungshilfsmittel schließen Polyquaternium-1, Polyquaternium-5, Polyquaternium-6, Polyquaternium-7, Polyquaternium-8, Polyquaternium-11, Polyquaternium-14, Polyquaternium-22, Polyquaternium-28, Polyquaternium-30, Polyquaternium-32 und Polyquaternium-33 ein (Bezeichnungen nach INCI).

Eine andere Gruppe geeigneter kationischer Polymere können Alkylamin-Epichlorhydrin-Polymere sein. Dies sind Reaktionsprodukte von Aminen und Oligoaminen mit Epichlorhydrin, wie beispielsweise in US 6,642,200 und US 6,551,986 beschrieben. Beispiele umfassen Dimethylamin-Epichlorhydrin-ethylendiamin, erhältlich unter dem Handelsnamen Cartafix(R) CB und Cartafix(R) TSF (Fa. Clariant).

Eine weitere Gruppe geeigneter synthetischer kationischer Polymere beinhaltet Polyamidoamine-Epichlorhydrin-Harze (PAE-Harze). Die häufigsten PAE-Harze sind die Kondensationsprodukte von Diethylentriamin mit Adipinsäure, die anschließend mit Epichlorhydrin umgesetzt werden. Derartige Harze sind beispielsweise erhältlich unter dem Handelsnamen Kymene™ (Fa. Hercules Inc.) oder Luresin™ (Fa. BASF). Die kationischen Polymere können ladungsneutralisierende Anionen enthalten, so dass das Gesamtpolymer unter Umgebungsbedingungen neutral ist. Beispiele für geeignete Gegenionen (zusätzlich zu Anionen, die während der Verwendung entstehen) umfassen Chlorid, Bromid, Sulfat, Methylsulfat, Sulfonat, Methylsulfonat, Carbonat, Bicarbonat, Formiat, Acetat, Citrat, Nitrat und Mischungen davon.

Das gewichtsmittlere Molekulargewicht des gegebenenfalls als Deponierungsmittel eingesetzten Polymers kann von etwa 500 Dalton bis etwa 5.000.000 Dalton, bevorzugt von etwa 1.000 Dalton bis etwa 2.000.000 Dalton, insbesondere bevorzugt von etwa 2.500 Dalton bis etwa 1.500.000 Dalton betragen, gemessen durch Größenausschluss-Chromatographie relativ zu Polyethylenoxiden als Standard via RI-Detektion. Besonders bevorzugt kann das Molekulargewicht des kationischen Polymers von etwa 500 Dalton bis etwa 37.500 Dalton betragen.

Beispiele für weitere Stoffe sind pH-Regulierungsmittel. pH-Regulierungsmittel können in einer solchen Menge in den erfindungsgemäßen Zusammensetzungen eingesetzt werden, dass der pH-Wert der erfindungsgemäßen Zusammensetzungen vorzugsweise zwischen 1 und 8, bevorzugt zwischen 1 und 6 und besonders bevorzugt zwischen 1,5 und 3,5, liegt. Einsetzbar sind hier sämtliche bekannten Säuren bzw. Laugen, sofern sich ihr Einsatz nicht aus anwendungstechnischen oder ökologischen Gründen bzw. aus Gründen des Verbraucherschutzes verbietet.

Beispiele für weitere Stoffe sind Farbstoffe, um den ästhetischen Eindruck der erfindungsgemäßen Zusammensetzungen zu verbessern. Bevorzugte Farbstoffe, deren Auswahl dem Fachmann keinerlei Schwierigkeit bereitet, besitzen eine hohe Lagerstabilität und Unempfindlichkeit gegenüber den übrigen Inhaltsstoffen der erfindungsgemäßen Zusammensetzungen und gegen Licht.

Beispiele für weitere Stoffe sind Schauminhibitoren, wie beispielsweise Seifen, Paraffine oder Silikonöle, die gegebenenfalls auf Trägermaterialien aufgebracht sein können.

Beispiele für weitere Stoffe sind geringe Mengen an nichtionischen Tensiden, um beispielsweise eine homogene Dispersion des Parfümöls zu gewährleisten.

Zur Herstellung der erfindungsgemäßen Zusammensetzungen wird in der Regel zunächst das kationische Tensid (B) aufgeschmolzen und die Schmelze in Wasser dispergiert. Anschließend wird das Carbamato-funktionalisierte Polydiorganosiloxan (A) in einer wässrigen Emulsion bzw. gegebenenfalls die weiteren Inhaltsstoffe wie beispielsweise der Elektrolyt, der Entschäumer und der Farbstoff zugemischt. Zum Schluss wird gegebenenfalls das Parfüm zugegeben.
Das Carbamato-funktionalisierte Polydiorganosiloxan (A) in einer wässrigen Emulsion kann auch vorgelegt und gegebenenfalls mit Wasser verdünnt werden, um anschließend darin das aufgeschmolzene kationische Tensid (B) zu dispergieren.

Die Carbamato-funktionalisierten Polydiorganosiloxane (A) werden vorzugsweise in Form ihrer wässrigen Emulsionen eingesetzt. Bei den wässrigen Emulsionen werden die Carbamato-funktionalisierten Polydiorganosiloxane (A) vorzugsweise in Mengen von mindestens 5 Gew.-%, bevorzugt mindestens 7 Gew.-%, insbesondere mindestens 10 Gew.-%, und vorzugsweise höchstens 70 Gew.-%, bevorzugt höchstens 50 Gew.-%, insbesondere höchstens 40 Gew.-%, jeweils bezogen auf das Gesamtgewicht der Emulsionen, eingesetzt.

Die Stabilisierung der wässrigen Emulsionen der Carbamato-funktionalisierten Polydiorganosiloxane (A) erfolgt unter Verwendung von Emulgatoren (E), dies können nicht-ionische Emulgatoren und/oder kationische Emulgatoren und/oder Schutzkolloide, insbesondere nicht-ionischen Emulgatoren oder eine Kombination aus nicht-ionischen und kationischen Emulgatoren sein.

Beispiele für nichtionische Emulgatoren sind:
1. Polyvinylalkohol, der noch 5 bis 50%, vorzugsweise 8 bis 20% Vinylacetateinheiten aufweist, mit einem Polymerisationsgrad von 500 bis 3000.
2. Alkylpolyglycolether, vorzugsweise solche mit 3 bis 40 EO-Einheiten und Alkylresten von 8 bis 20 C-Atomen.
3. Alkylarylpolyglycolether, vorzugsweise solche mit 5 bis 40 EO-Einheiten und 8 bis 20 C-Atomen in den Alkyl- und Arylresten.
4. Ethylenoxid/Propylenoxid(EO/PO)-Blockcopolymere, vorzugsweise solche mit 8 bis 40 EO- bzw. PO-Einheiten.
5. Additionsprodukte von Alkylaminen mit Alkylresten von 8 bis 22 C-Atomen mit Ethylenoxid oder Propylenoxid.
6. Fettsäuren mit 6 bis 24 C-Atomen.
7. Alkylpolyglykoside der allgemeinen Formel R*-O-Z_{O}, worin R* einen linearen oder verzweigten, gesättigten oder ungesättigten Alkylrest mit im Mittel 8-24 C-Atomen und Z_{O} einen Oligoglykosidrest mit im Mittel o = 1-10 Hexose- oder Pentoseeinheiten oder Gemischen davon bedeuten.
8. Naturstoffe und deren Derivate, wie Lecithin, Lanolin, Saponine, Cellulose; Cellulosealkylether und Carboxyalkylcellulosen, deren Alkylgruppen jeweils bis zu 4 Kohlenstoffatome besitzen.
9. Polare Gruppen, enthaltend insbesondere die Elemente O, N, C, S, P, Si, enthaltende lineare Organo(poly)siloxane, insbesondere solche mit Alkoxygruppen mit bis zu 24 C-Atomen und/oder bis zu 40 EO- und/oder PO-Gruppen.

Beispiele für kationische Emulgatoren sind:
10. Salze von primären, sekundären und tertiären Fettaminen mit 8 bis 24 C-Atomen mit Essigsäure, Schwefelsäure, Salzsäure und Phosphorsäuren.
11. Alkylpyridinium-, Alkylimidazolinium- und Alkyloxazoliniumsalze, insbesondere solche, deren Alkylkette bis zu 18 C-Atome besitzt, speziell die Halogenide, Sulfate, Phosphate und Acetate.
12. Kationisches Tenside der Formel (B1) (Quartäre Ammonium Tenside) und der Formel (B2) (Ester-/Amido-haltige quartäre Ammonium Tenside), wie zuvor beschrieben.

Als ampholytische Emulgatoren eignen sich besonders:
13. Langkettig substituierte Aminosäuren, wie N-Alkyl-di-(aminoethyl-)glycin oder N-Alkyl-2-aminopropionsäuresalze.
14. Betaine, wie N-(3-Acylamidopropyl)-N,N-dimethylammoniumsalze mit einem C₈-C₁₈-Acylrest und Alkylimidazolium-Betaine.

Bevorzugt als Emulgatoren sind nichtionische Emulgatoren, insbesondere die vorstehend unter 2. aufgeführten Alkylpolyglycolether sowie kationische Emulgatoren, insbesondere die vorstehend unter 12. aufgeführten kationischen Tenside. Die Emulgatoren (E) können allein oder in Form eines Gemisches zweier oder mehrerer o.g. Emulgatoren verwendet werden, sie können in reiner Form oder als Lösungen eines oder mehrerer Emulgatoren in Wasser oder organischen Lösungsmitteln eingesetzt werden.

Bei den wässrigen Emulsionen der erfindungsgemäßen Carbamato-funktionalisierten Polydiorganosiloxane (A) werden die Emulgatoren (E) vorzugsweise in Mengen von mindestens 1 Gew.-%, bevorzugt mindestens 2 Gew.-%, insbesondere mindestens 3 Gew.-%, und vorzugsweise höchstens 25 Gew.-%, bevorzugt höchstens 20 Gew. %, insbesondere höchstens 15 Gew.-%, jeweils bezogen auf das Gesamtgewicht der Emulsionen, eingesetzt.

Ferner können zur Verkleinerung der Teilchengröße und zur Verringerung der benötigten Menge an Emulgatoren (E) in den wässrigen Emulsionen der Carbamato-funktionalisierten Polydiorganosiloxane (A) Cotenside (Co-E) eingesetzt werden.

Unter Cotensiden (Co-E) versteht man polare Verbindungen mittlerer Molmasse, wie Alkohole der Molekülgröße C₃ bis C₈, geeignete Di- und Polyole, Amine, Ester und Ketone.

Sie stammen beispielsweise aus der Gruppe der ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen wie 1-Butanol, 2-Butanol oder 2-Methyl-2-propanol, Pentanole wie 1-Pentanol, 2-Pentanol oder 3-Pentanol, Hexanole wie 1-Hexanol, 2-Hexanol oder 3-Hexanol, Heptanole wie 1-Heptanol, 2-Heptanol, 3-Heptanol oder 4-Heptanol, Octanole wie 1-Octanol, 2-Octanol, 3-Octanol oder 4-Octanol, Glycol, Propandiol, Butandiole wie 1,2-Butandiol oder 1,3-Butandiol, Hexandiole wie 1,2-Hexandiol oder 2-Methylpentan-2,4-diol, Octandiole wie 2-Ethylhexan-1,3-diol oder 1,2-Octandiol, Glycerin, Diglykol, Propyl- oder Butyldiglykol, Ethylenglykolmethylether, Ethylenglykolethylether, Ethylenglykolpropylether, Ethylenglykolmono-n-butylether, Diethylenglykolmethylether, Diethylenglykolethylether, Diethylenglykolmono-n-butylether, Propylenglykolmethylether, Propylenglykolethylether, Propylenglykolpropylether, Propylenglykol-n-butylether, Propylen-glykol-tert-butylether, Methoxytriglykol, Ethoxytriglykol, Butoxytriglykol, Dipropylenglykolmonomethylether, Dipropylenglykolmonoethylether, 1-Butoxyethoxy-2-propanol oder 3-Methyl-3-methoxybutanol, 1-Aminobutan, 2-Aminobutan, 2-Amino-2-methylpropan, 1-Aminopentan, 2-Aminopentan, 1-Aminohexan, 1-Aminoheptan und 1-Aminooctan; Ethyl-, Propyl-, Isopropyl-, Butyl-, Isobutyl-, Pentyl-, Isopentyl- und Hexylacetat; Methyl-, Ethyl- und tert.-Butylpropionat; Methyl-, Ethyl-, Propyl- und Butylbutyrat; 2-Butanon, 2-Pentanon, 3-Pentanon, 4-Methyl-2-pentanon, 2-Hexanon, 3-Hexanon, 2-Heptanon, 3-Heptanon, 4-Heptanon, 5-Methyl-3-Heptanon, 2-Octanon und 3-Octanon sowie Mischungen dieser Cotenside.
Beispiele für bevorzugte Cotenside (Co-E) sind 1-Alkanole der vorstehend aufgeführten Beispiele mit C₅- bis C₈-Ketten, Alkandiole der vorstehend aufgeführten Beispiele mit C₄- bis C₈-Kette, Glycerin, Propyl-, Butyl- und Pentylacetat, 2-Pentanon sowie die oben aufgeführten Ethylenglykol-, Propylenglykol-, Dipropylenglykol- oder Diethylenglykolmonoalkylether.

Bei den erfindungsgemäßen wässrigen Emulsion der Carbamato-funktionalisierten Polydiorganosiloxane (A) werden die Co-Emulgatoren (Co-E) vorzugsweise in Mengen von mindestens 1 Gew.-%, bevorzugt mindestens 2 Gew.-%, insbesondere mindestens 3 Gew.-%, und vorzugsweise höchstens 15 Gew.-%, bevorzugt höchstens 10 Gew.-%, insbesondere höchstens 7 Gew.-%, jeweils bezogen auf das Gesamtgewicht der Emulsionen, eingesetzt.

Die erfindungsgemäßen wässrigen Emulsionen der Carbamato-funktionalisierten Polydiorganosiloxane (A) können Säuren (F) zur Einstellung eines gewünschten pH-Werts oder zur Bildung von Säureadditionssalzen mit den Amino-haltigen Resten (Y) der Carbamato-funktionalisierten Polydiorganosiloxane (A) enthalten.

Beispiele für Mineralsäuren, die sich beispielsweise mit den vorstehend genannten Amino-haltigen Resten (Y) umsetzen lassen, sind Salzsäure, Perchlorsäure, Schwefelsäure, schweflige Säure, Salpetersäure, salpetrige Säure, Flusssäure, Phosphor-, Diphosphor- und Polyphosphorsäuren. Beispiele für geeignete Carbonsäuren sind Ameisensäure, Essigsäure, Propionsäure, Butansäuren, Citronensäure, Trichloressigsäure, Dichloressigsäure und Chloressigsäure, Trifluoressigsäure, Cyanessigsäure, Phenylessigsäure, Benzoesäure, m- und p-Nitrobenzoesäure, Oxalsäure, Malonsäure und Milchsäure.

Besonders bevorzugt sind Essigsäure und Ameisensäure.

Die erfindungsgemäßen wässrigen Emulsionen der Carbamato-funktionalisierten Polydiorganosiloxane (A) können neben Emulgator (E), gegebenenfalls Cotensid (Co-E) und gegebenenfalls Säure (F) noch Zusatzstoffe (H) enthalten. Beispiele für Zusatzstoffe (H) sind insbesondere Bakterizide, Fungizide, Algizide, Mikrobiozide, Duftstoffe, Korrosionsinhibitoren, Farbstoffe, Pigmente, Verdickungsmittel und Füllstoffe.

Der Emulgiervorgang zur Herstellung der erfindungsgemäßen wässrigen Emulsion der Carbamato-funktionalisierten Polydiorganosiloxane (A) wird vorzugsweise bei Temperaturen von mindestens 10°C, bevorzugt mindestens 15°C, und vorzugsweise höchstens 80°C, bevorzugt höchstens 70°C, durchgeführt.

Die Temperaturerhöhung kommt vorzugsweise durch den Eintrag mechanischer Scherenergie, die für den Emulgierprozess benötigt wird, zustande. Die Temperaturerhöhung wird nicht zur Beschleunigung eines chemischen Prozesses benötigt. Weiterhin wird das erfindungsgemäße Verfahren vorzugsweise beim Druck der umgebenden Atmosphäre durchgeführt, kann aber auch bei höheren oder niederen Drücken durchgeführt werden.

Die Herstellung der erfindungsgemäßen Emulsionen der Carbamato-funktionalisierten Polydiorganosiloxane (A) erfolgt durch intensives Mischen des Carbamato-funktionalisierten Polydiorganosiloxans (A) in wässrigem Medium mit dem Emulgator (E), gegebenenfalls dem Coemulgator (Co-E), gegebenenfalls Säuren (F) und gegebenenfalls Zusatzstoffen (H) miteinander. Es werden stabile Emulsionen gebildet. Dadurch liegen die Carbamato-funktionalisierten Polydiorganosiloxane (A) in fein verteilter Form vor.

Die Herstellung der erfindungsgemäßen Emulsionen der Carbamato-funktionalisierten Polydiorganosiloxane (A) kann diskontinuierlich oder kontinuierlich erfolgen.

Technologien zur Herstellung von Emulsionen von Organopolysiloxanen sind bekannt. So kann das intensive Mischen und Dispergieren in Rotor-Stator-Rührvorrichtungen, Kolloidmühlen, Hochdruckhomogenisatoren, Mikrokanälen, Membranen, Strahldüsen und ähnlichem, oder mittels Ultraschall erfolgen. Homogenisiergeräte und Verfahren sind z.B. in Ullmann's Encyclopedia of Industrial Chemistry, CD-ROM-Ausgabe 2003, Wiley-VCH Verlag, unter dem Stichwort "Emulsions" beschrieben.

Die Art der Mischung der Komponenten, die zur Herstellung der erfindungsgemäßen Emulsionen gebraucht werden, ist nicht sehr kritisch und kann in verschiedener Reihenfolge ausgeübt werden. In Abhängigkeit von den Komponenten (A), (E), gegebenenfalls (Co-E), gegebenenfalls (F) und gegebenenfalls (H) können sich aber bevorzugte Vorgehensweisen ergeben, die im Einzelfall geprüft werden sollten.

Es können z.B. die Komponente (A) und gegebenenfalls die Säure (F) vorgelegt werden, daraufhin die Emulgatoren (E) und gegebenenfalls die Coemulgatoren (Co-E) zugefügt und danach das Dispersionsmittel und gegebenenfalls Zusatzstoffe (H) eingearbeitet werden. In vielen Fällen hat sich gezeigt, dass es vorteilhaft ist, Emulgatoren (E), gegebenenfalls Coemulgatoren (Co-E) und gegebenenfalls Säure (F) mit einem Teil des Dispersionsmittels Wasser in der Emulgierapparatur vorzulegen und in diese erhaltene Mischung Komponente (A) und die weiteren Komponenten einzuarbeiten.

Bei dem erfindungsgemäßen Verfahren wird das Dispersionsmittel Wasser in Mengen von vorzugsweise mindestens 1 Gew.-%, bevorzugt mindestens 5 Gew.-%, insbesondere mindestens 10 Gew.-%, und vorzugsweise höchstens 99 Gew.-%, bevorzugt höchstens 95 Gew.-%, insbesondere höchstens 90 Gew.-%, jeweils bezogen auf das Gesamtgewicht aller Inhaltsstoffe der Emulsion eingesetzt.

Die erfindungsgemäßen Zusammensetzungen aus Carbamato-funktionalisierten Polydiorganosiloxanen (A) und kationischen Tensiden (B) finden Verwendung als Wirkstoffzusammensetzungen in Produkten für die Pflege und Reinigung.

Unter dem Begriff "Wirkstoff" wird hier eine Substanz verstanden, die den Zweck erfüllt,
(a) einen Gegenstand zu pflegen, das heißt einen Gegenstand in seiner ursprünglichen Form zu erhalten, die Auswirkungen äußerer Einflüsse (z.B. Zeit, Licht, Temperatur, Druck, Verschmutzung, chemische Reaktion mit anderen, mit dem Gegenstand in Kontakt tretenden reaktiven Verbindungen) wie beispielsweise Altern, Verschmutzen, Materialermüdung, Ausbleichen, zu mindern oder zu vermeiden oder sogar gewünschte positive Eigenschaften des Gegenstandes zu verbessern,
   und
(b) einen Gegenstand zu reinigen, das heißt die Entfernung von Verunreinigungen als Folge der Benutzung des Gegenstands zu bewirken oder zu unterstützen.

Handelt es sich bei der Pflege eines Gegenstands um die Behandlung von Textilfasern bzw. -geweben können mit den erfindungsgemäßen Zusammensetzungen beispielsweise folgende vorteilhafte Effekte erzielt werden:
Das Erzielen einer deutlich verbesserten Weichheit der Fasern/Gewebe nach dem Waschen, die Reduktion der Faltenbildung des Gewebes während der Spül- und Trocknungsstufen, die Reduktion des Auftretens von Falten oder Knicken vor dem Bügeln, die Verringerung der Kraft, die zum Bügeln des Gewebes benötigt wird, der Schutz vor Faltenbildung während des Benutzens, der Erhalt der Form des textilen Flächengewebes beim Waschen, bei der Pflege und bei der Benutzung, die Verbesserung der Benetzbarkeit der Fasern/Gewebe, die Reduzierung des Effekts des Pillings (also der Knötchen- oder Fusselbildung) bei Textilgeweben, das Unterbinden des auftretenden Effekts der Trockenstarre bei trocknender Wäsche, das Erzielen einer größeren Elastizität bei Fasern/Geweben, das Erzielen eines verbesserten Glanzes bei Fasern oder das Reduzieren des Verblassens der Farben bei Fasern/Geweben.

In diesem Zusammenhang sind unter Produkten für die Pflege und Reinigung folgende Zusammensetzungen oder Formulierungen zu verstehen:
- Formulierungen, die man in Haushalt und Industrie einsetzt, zur Pflege und Reinigung von Oberflächen, wie beispielsweise Fasern, Leder, Stoffe, Holz, Glas, Keramik, Fliesen, Linoleum, Kunststoff.
   Beispiele für Mittel zur Reinigung und Pflege solcher Oberflächen sind Waschmittel (Vollwaschmittel, Colorwaschmittel, Weichspüler etc.), Geschirrspülmittel, Maschinengeschirrspülmittel, Klarspüler, Neutralreiniger, Fensterreinigungsmittel, Allzweckreiniger, Glasreiniger, Sanitärreiniger, WC-Reiniger, Teppichreiniger, Autopflegemittel.
- Formulierungen, die man zur Behandlung keratinischer Fasern, wie Haaren einsetzt. Vorzugsweise werden die kosmetischen Zusammensetzungen zur Reinigung und Pflege von Haaren verwendet. Beispiele für Mittel zur Reinigung und Pflege von Haaren sind Haar-Shampoos, Haar-Spülungen (Rinse-Off Conditioner), Haar-Kuren, Haar-Masken, Haar-Seren, HaarSchäume, Haar-Stylingsprays, Haar-Cremes, Haar-Gele, Haaröle, Haar-Spitzenfluide und Haar-Färbemittel.

Gegenstand der Erfindung sind daher Wasch- und Reinigungsmittel, insbesondere Weichspülmittel, enthaltend die erfindungsgemäßen Zusammensetzungen.

Die erfindungsgemäßen Zusammensetzungen können entweder direkt als Wasch- und Reinigungsmittel, insbesondere Weichspülmittel, Verwendung finden oder sie sind in den Wasch- und Reinigungsmittel, insbesondere Weichspülmittel, in Mengen von vorzugsweise 10 bis 90 Gew.-% enthalten.

Gegenstand der Erfindung ist daher ein Verfahren zur Pflege und Reinigung von Fasern, insbesondere Textilfasern und Textilgeweben, mit den erfindungsgemäßen Zusammensetzungen oder mit den Wasch- und Reinigungsmitteln enthaltend die erfindungsgemäßen Zusammensetzungen.

Gegenstand der Erfindung sind weiterhin kosmetische Formulierungen, insbesondere Haarbehandlungsmittel, enthaltend die erfindungsgemäßen Zusammensetzungen.

Die erfindungsgemäßen Zusammensetzungen können entweder direkt in kosmetischen Formulierungen, insbesondere Haarbehandlungsmitteln, Verwendung finden oder sie sind in kosmetischen Formulierungen, insbesondere Haarbehandlungsmitteln, in Mengen von vorzugsweise 10 bis 90 Gew.-% enthalten.

Gegenstand der Erfindung ist daher ein Verfahren zur Pflege und Reinigung von Haaren mit den erfindungsgemäßen Zusammensetzungen oder mit den kosmetischen Formulierungen enthaltend die erfindungsgemäßen Zusammensetzungen.

In den nachfolgenden Beispielen beziehen sich alle Angaben von Teilen und Prozentsätzen, soweit nicht anders angegeben, auf das Gewicht.
Sofern nicht anders angegeben, werden die folgenden Beispiele bei einem Druck der umgebenden Atmosphäre, also bei etwa 1000 hPa, und bei Raumtemperatur, also etwa 20°C bzw. einer Temperatur, die sich beim Zusammengeben der Reaktanten bei Raumtemperatur ohne zusätzliche Heizung oder Kühlung einstellt, durchgeführt.

Die Viskositäten wurden an einem Rheometer "MCR 302" der Fa. Anton Paar nach DIN EN ISO 3219: 1994 und DIN 53019 gemessen, wobei ein Kegel-Platte-System (Kegel CP50-2) mit einem Öffnungswinkel von 2° verwendet wurde. Die Kalibrierung des Gerätes erfolgte mit Normalöl 10000 der Physikalisch-Technischen Bundesanstalt. Die Messtemperatur beträgt 25,00°C +/- 0,05°C, die Messzeit 3 min. Die Viskositätsangabe stellt den arithmetischen Mittelwert von drei unabhängig durchgeführten Einzelmessungen dar. Die Messunsicherheit der dynamischen Viskosität beträgt 1,5 %. Der Schergeschwindigkeitsgradient wurde in Abhängigkeit von der Viskosität gewählt und wird für jede Viskositätsangabe separat ausgewiesen.

Die Aminzahl gibt an, wieviel mmol KOH einem Gramm der zu bestimmenden Substanz äquivalent sind. Die Bestimmung der Aminzahl erfolgt nach DIN 16945-Version 1989-03.

1H-NMR-Spektren werden als Lösung in CDCl₃ an einem Bruker Avance 500-NMR-Spektrometer (5 mm selektiver 1H-NMR-Probenkopf) mit einer Messfrequenz 500,13 MHz aufgenommen.
Die Auswertung erfolgt wie dem Fachmann bekannt und in folgender Literatur beschrieben: "Über die ¹H-, ¹³C- und ²⁹Si-NMR chemischen Verschiebungen einiger linearer, verzweigter und cyclischer Methyl-Siloxan-Verbindungen", G. Engelhardt, H. Jancke; J. Organometal. Chem. 28 (1971), 293-300; "Chapter 8 - NMR spectroscopy of organosilicon compounds", Elizabeth A. Williams, The Chemistry of Organic Silicon Compounds, 1989 John Wiley and Sons Ltd, 511-533.

Die Partikelgrößen wurden an einem Partikelgrößenmessgerät Zetasizer Nano-S, Fa. Malvern, Software Version 6.01 mittels dynamischer Lichtstreuung (Messmethode nach Mie) bestimmt. Dazu wurden die Emulsionen mit gefilterten und entgasten Wasser auf 0,5 Gew.-% verdünnt. Die angegebenen Werte beziehen sich immer auf den Wert D(50). D(50) ist als volumengemittelter Partikeldurchmesser zu verstehen, bei dem 50% aller gemessenen Partikel einen volumengemittelten Durchmesser kleiner als der ausgewiesene Wert D(50) aufweisen.

### Beispiele:

### Beispiel 1: Carbamato-funktionalisiertes Polydimethylsiloxan 1:

300,0 g eines gemischt Hydroxy-/Methoxy-terminierten Copolymers aus Aminoethylaminopropylmethylsiloxan- und Dimethylsiloxan-Einheiten der Viskosität 1057 mPas (bei 25°C und bei einer Scherrate von 10 1/s) und der Aminzahl von 0,295 mmol/g werden in einem 500 ml Dreihalskolben unter Stickstoffatmosphäre vorgelegt und mit 4,52 g Propylencarbonat versetzt. Die Reaktionsmischung wird langsam auf 50°C aufgeheizt und zwei Stunden bei dieser Temperatur gerührt. Nach Abkühlen erhält man ein klares Produkt **1** der Viskosität 3173 mPas (bei 25°C und bei einer Scherrate von 10 1/s). Das ¹H-NMR-Spektrum zeigt einen Funktionalisierungsgrad bzgl. Carbamat von 46 % aller zur Verfügung stehenden Aminogruppen.

### Beispiel 2: Carbamato-funktionalisiertes Polydimethylsiloxan 2:

715,6 g eines gemischt Hydroxy-/Methoxy-terminierten Copolymers aus Aminoethylaminopropylmethylsiloxan- und Dimethylsiloxan-Einheiten der Viskosität 982 mPas (bei 25°C und bei einer Scherrate von 10 1/s) und der Aminzahl von 0,287 mmol/g werden in einem 1000 ml Dreihalskolben unter Stickstoffatmosphäre vorgelegt und mit 5,00 g Propylencarbonat versetzt. Die Reaktionsmischung wird langsam auf 50°C aufgeheizt und zwei Stunden bei dieser Temperatur gerührt. Nach Abkühlen erhält man ein klares Produkt **2** der Viskosität 2280 mPas (bei 25°C und bei einer Scherrate von 10 1/s). Das ¹H-NMR-Spektrum zeigt einen Funktionalisierungsgrad bzgl. Carbamat von 23 % aller zur Verfügung stehenden Aminogruppen.

### Beispiel 3: Carbamato-funktionalisiertes Polydimethylsiloxan 3:

300,0 g eines Trimethylsilyl-terminierten Copolymers aus Aminoethylaminopropylmethylsiloxan- und Dimethylsiloxan-Einheiten der Viskosität 1111 mPas (bei 25°C und bei einer Scherrate von 10 1/s) und der Aminzahl von 0,595 mmol/g und 34,2 g Dipropylenglycolmonobutylether (käuflich erwerblich unter dem Namen DOWANOL® DPnB von der Fa. Aldrich) werden in einem 500 ml Dreihalskolben unter Stickstoffatmosphäre vorgelegt und mit 9,20 g Propylencarbonat versetzt. Die Reaktionsmischung wird langsam auf 50°C aufgeheizt und zwei Stunden bei dieser Temperatur gerührt. Nach Abkühlen erhält man ein klares Produkt **3** der Viskosität 1570 mPas (bei 25°C und bei einer Scherrate von 10 1/s). Das ¹H-NMR-Spektrum zeigt einen Funktionalisierungsgrad bzgl. Carbamat von 48 % aller zur Verfügung stehenden Aminogruppen.

### Beispiel 4: Emulsion E1 aus Carbamato-funktionalisiertem Polydimethylsiloxan 1:

In einem Ultra-Turrax T 50 Emulgiergerät (Fa. Janke & Kunkel/IKA) werden 4,0 g Isotridecyloctaethoxylat, käuflich erwerblich unter dem Handelsnamen Lutensol TO 8 (Fa. BASF), 2,0 g Isotridecylpentaethoxylat, käuflich erwerblich unter dem Handelsnamen Lutensol TO 5 (Fa. BASF) und 6,9 g vollentsalztes Wasser vorgelegt. 33,0 g des Carbamato-funktionalisierten Produkts **1** werden in fünf Portionen unter hoher Scherung von 6000 bis 8000 UmP zugegeben, so dass eine relativ feste steife Phase als Voremulsion resultiert. Es wird mit 0,8 g konzentrierter Essigsäure versetzt und mit 60,2 g vollentsalztem Wasser portionsweise unter geringer Scherung zur gewünschten Emulsion verdünnt. Es resultiert eine milchig-weiße Emulsion mit einer durchschnittlichen Partikelgröße von 61 nm. Die Emulsion ist auch nach 6-monatiger Lagerung bei Raumtemperatur homogen und stabil.

### Beispiel 5: Emulsion E2 aus dem Carbamato-funktionalisierten Polydimethylsiloxanen 2:

Emulsion **E2** wird nach der gleichen Vorschrift hergestellt wie in Beispiel 3 beschrieben, aber mit dem Carbamato-funktionalisierten Polydimethylsiloxanen **2** aus Beispiel 2. Es resultiert eine milchig-weiße Emulsion mit einer durchschnittlichen Partikelgröße von 145 nm. Die Emulsion ist auch nach 6-monatiger Lagerung bei Raumtemperatur homogen und stabil.

### Beispiel 6: Emulsion E3 aus dem Carbamato-funktionalisierten Polydimethylsiloxanen 3:

Emulsion **E3** wird nach der gleichen Vorschrift hergestellt wie in Beispiel 4 beschrieben, aber mit dem Carbamato-funktionalisierten Polydimethylsiloxanen **3** aus Beispiel 3. Es resultiert eine milchig-weiße Emulsion mit einer durchschnittlichen Partikelgröße von 157 nm. Die Emulsion ist auch nach 6-monatiger Lagerung bei Raumtemperatur homogen und stabil.

### Beispiel 7: Mikroemulsion E4 aus Carbamato-funktionalisiertem Polydimethylsiloxan 2:

In einem Ultra-Turrax T 50 Emulgiergerät (Fa. Janke & Kunkel/IKA) werden 5,5 g Isotridecylpentaethoxylat, käuflich erwerblich unter dem Handelsnamen Lutensol TO 5 (Fa. BASF), 2,50 g Diethylenglykolmonobutylether, käuflich erwerblich unter dem Handelsnamen Butyldiglykol (Fa. Brenntag) und 8,0 g vollentsalztes Wasser vorgelegt. 16,5 g des Carbamato-funktionalisierten Produkts **2** werden in fünf Portionen unter hoher Scherung von 6000 bis 8000 UpM zugegeben, so dass eine relativ feste steife Phase als Voremulsion resultiert. Es wird mit 0,1 g konzentrierter Essigsäure versetzt und mit 75,3 g vollentsalztem Wasser portionsweise unter geringer Scherung zur gewünschten Emulsion verdünnt. Es resultiert eine klare Emulsion **E4** mit einer durchschnittlichen Partikelgröße von 19 nm. Die Emulsion ist auch nach 6-monatiger Lagerung bei Raumtemperatur homogen und stabil.

### Beispiel 8: Kationische Emulsionen E5 aus Carbamato-funktionalisiertem Polydimethylsiloxan 2:

In einem Ultra-Turrax T 50 Emulgiergerät (Fa. Janke & Kunkel/IKA) werden 10,4 g Isotridecyloctaethoxylat, käuflich erwerblich unter dem Handelsnamen Lutensol TO 8 (Fa. BASF), 5,2 g Isotridecylpentaethoxylat, käuflich erwerblich unter dem Handelsnamen Lutensol TO 5 (Fa. BASF), 0,54 g N,N-bis[Ethyl(tallowate)]-N-(2-hydroxyethyl)-N-methylammonium-methylsulfat (90%ige ethanolische Lösung), käuflich erwerblich unter dem Handelsnamen Stepantex® VK90 (Fa. Stepan) und 10,7 g vollentsalztes Wasser vorgelegt. 69,4 g des Carbamato-funktionalisierten Produkts **2** werden in zwei Portionen unter hoher Scherung von 4000 bis 6000 UpM zugegeben, so dass eine pastöse Voremulsion resultiert. Es wird mit 0,4 g konzentrierter Essigsäure versetzt und mit 102,9 g vollentsalztem Wasser portionsweise unter geringer Scherung zur gewünschten Emulsion verdünnt. Es resultiert eine opaleszierende Emulsion **E5** mit einer durchschnittlichen Partikelgröße von 90 nm. Die Emulsion ist auch nach 6-monatiger Lagerung bei Raumtemperatur homogen und stabil.

### Beispiel 9: Kationische Emulsionen E6 aus Carbamato-funktionalisiertem Polydimethylsiloxan 2:

In einem Ultra-Turrax T 50 Emulgiergerät (Fa. Janke & Kunkel/IKA) werden 22,0 g Isotridecylpentaethoxylat, käuflich erwerblich unter dem Handelsnamen Lutensol TO 5 (Fa. BASF), 10,0 g Diethylenglykol-monobutylether, käuflich erwerblich unter dem Handelsnamen Butyldiglykol (Fa. Brenntag), 1,50 g N,N-bis[Ethyl(tallowate)]-N-(2-hydroxyethyl)-N-methylammoniummethylsulfat (90%ige ethanolische Lösung), käuflich erwerblich unter dem Handelsnamen Stepantex® VK90 (Fa. Stepan) und 22,0 g vollentsalztes Wasser vorgelegt. 66,0 g des Carbamato-funktionalisierten Produkts **2** werden in zwei Portionen unter hoher Scherung von 4000 bis 6000 UpM zugegeben, so dass eine lotionsartige Voremulsion resultiert. Es wird mit 77,58 g vollentsalztem Wasser portionsweise unter geringer Scherung zur gewünschten Emulsion verdünnt, der dann abschließend 0,5 g 80%ige Essigsäure sowie 0,40 g Natriumacetat zugesetzt werden. Es resultiert eine klare Emulsion **E6** mit einer durchschnittlichen Partikelgröße von 21 nm. Die Emulsion ist auch nach 6-monatiger Lagerung bei Raumtemperatur homogen und stabil.

### Beispiel 10:

Wässrige Formulierungen **F1** - **F3, F7** und **F9** (erfindungsgemäß) enthaltend
(A) Carbamato-funktionalisierte Polydiorganosiloxane und
(B) kationische Tenside.

Wässrige Formulierung **F4** (nicht erfindungsgemäß) enthaltend (B) kationische Tenside ohne Komponente (A).

Wässrige Formulierungen **F5** und **F6** (nicht erfindungsgemäß) enthaltend
(A) Carbamato-funktionalisierte Polydiorganosiloxane ohne Komponente (B).

Die Anwendungstests auf Weichgriff, Tropfeneinsinkzeit, Easy Ironing und den Knittererholungswinkel erfolgten unter Verwendung folgender in Tabelle 1 beschriebener wässriger Formulierungen:

**Tabelle 1: Wässrige Formulierungen F1 bis F8**

| | Emulsion aus Carbamato-funktionalisiertem Polydimethylsiloxan | Kationisches Tensid **T1** ***) | Wasser | Elektrolyte, Parfüms, Farbstoffe, Konservierungsmittel |
|---|---|---|---|---|
| **F1** *) | 2,1 g **E2** | 2,1 g | 32,3 g | 0,7 g |
| **F2** *) | 2,1 g **E3** | 2,1 g | 32,3 g | 0,7 g |
| **F3** *) | 1,1 g **E1** | 2,3 g | 20,7 g | 0,5 g |
| **F4** **) | - | 3,5 g | 31,2 g | 0,3 g |
| **F5** **) | 17,6 g **E2** | - | 19,5 g | 0,3 g |
| **F6** **) | 17,6 g **E3** | - | 19,5 g | 0,3 g |
| **F7** *) | 2,1 g **E4** | 2,1 g | 32,3 g | 0,7 g |
| **F8** *) | 2,1 g **E5** | 2,1 g | 32,3 g | 0,7 g |

| | | | | |
|---|---|---|---|---|
| *) erfindungsgemäß **) nicht erfindungsgemäß ***) **T1:** N,N-bis[Ethyl(tallowate)]-N-(2-hydroxyethyl)-N-methylammoniummethylsulfat (90%ige ethanolische Lösung), käuflich erwerblich unter dem Handelsnamen Stepantex® VK90 (Fa. Stepan) (Tensid gemäß der Formel (B2-1)) | | | | |

Die (erfindungsgemäßen wie auch nicht erfindungsgemäßen) Formulierungen werden dadurch hergestellt, dass man das Wasser auf 50°C erhitzt. Das zuvor bei 50°C aufgeschmolzene und intensiv gerührte kationische Tensid wird bei dieser Temperatur unter intensivem Rühren (mit Hilfe eines Rührwerks der Marke IKA Eurostar Power basic mit Flügelrührer) hinzugefügt. Es wird weitergerührt, bis eine homogene Mischung resultiert. Die Mischung wird auf 30°C abgekühlt und die Emulsion aus Carbamato-funktionalisiertem Polydimethylsiloxan sowie weitere Inhaltsstoffe werden hinzugefügt.

### Beispiel 11:

In analoger Weise wie in Beispiel 10 beschrieben, wurden statt des kationischen Tensids (**T1**) N,N-bis[Ethyl(tallowate)]-N-(2-hydroxyethyl)-N-methylammoniummethylsulfats folgende kationischen Tenside verwendet:
- Cetyltrimethylammoniumchlorid, (29%ige wässrige Lösung), käuflich erwerblich unter dem Namen Genamin CTAC (Fa. Clariant) (kationisches Tensid **T2**), (Tensid gemäß der Formel (B1-1)),
- N,N-Distearyldimethylammoniumchlorid (95%ig), käuflich erwerblich unter dem Handelsnamen AROSURF® TA 100 (Fa. Evonik) (kationisches Tensid **T3**).
   (Tensid gemäß der Formel (B1-2))
- N,N-di(Alkylcarboxyethyl)-N-hydroxyethyl-N-methylammonium methyl sulfate (Alkyl = C16-Alkyl,C18-Alkyl, C18-Alkylen) (86%ige ethanolische Lösung), käuflich erwerblich unter dem Handelsnamen REWOQUAT® WE 45 (Fa. Evonik) (kationisches Tensid **T4**),
   (Tensid gemäß der Formel (B2-1))
- 1-Methyl-2-talloylamidoethyl-2-talloyl-imidazolinium methyl sulfate (90%ige Lösung in Isopropanol), käuflich erwerblich unter dem Handelsnamen ACCOSOFT® 808-90 (Fa. Stepan) (kationisches Tensid **T5**)
   (Tensid gemäß der Formel (B2-3))
- 1-Methyl-2-norstearyl-3-stearinsäure-amidoethyl-imidazolinium-methosulfat (75%ige Lösung in Isopropanol), käuflich erwerblich unter dem Handelsnamen REWOQUAT® W 75 (Fa. Evonik) (kationisches Tensid **T6**,)
   (Tensid gemäß der Formel (B2-3))
- N,N-(Talloylamidoethyl)-N-polyethoxyethyl-N-methylammonium methosulfate (90%ige Lösung), käuflich erwerblich unter dem Handelsnamen Varisoft(R) 222 LM (90%) (Fa. Evonik) (kationisches Tensid **T7**)**.**
   (Tensid gemäß der Formel (B2-4))

Die angegebenen kationischen Tenside **T2** bis **T7** wurden im Falle der nicht erfindungsgemäßen Formulierungen als 10%ige Formulierungen (bezogen auf den Festgehalt) formuliert (**F9** bis **F14)** oder im Falle der erfindungsgemäßen Formulierungen als 5%ige Formulierungen (bezogen auf den Festgehalt) zusammen mit 5% der Emulsion **E2** aus Carbamato-funktionalisiertem Polydimethylsiloxan formuliert **(F15** bis **F20**)**.** Die Formulierungen **F9** bis **F14** sowie **F15** bis **F20** sind in Tabelle 2 zusammengefasst.

**Tabelle 2: Wässrige Formulierungen F9 - F14 (nicht erfindungsgemäß) und F15 - F20 (erfindungsgemäß)**

| | Emulsion aus Carbamato-funktionalisiertem Polydimethylsiloxan | Kationisches Tensid |
|---|---|---|
| **F9** **) | - | **T2,** 10 Gew.-% |
| **F10** **) | - | **T3,** 10 Gew.-% |
| **F11** **) | - | **T4,** 10 Gew.-% |
| **F12** **) | - | **T5,** 10 Gew.-% |
| **F13** **) | - | **T6,** 10 Gew.-% |
| **F14** **) | - | **T7,** 10 Gew.-% |
| **F15** *) | **E2,** 5 Gew.-% | **T2,** 5 Gew.-% |
| **F16** *) | **E2,** 5 Gew.-% | **T3,** 5 Gew.-% |
| **F17** *) | **E2,** 5 Gew.-% | **T4,** 5 Gew.-% |
| **F18** *) | **E2,** 5 Gew.-% | **T5,** 5 Gew.-% |
| **F19** *) | **E2,** 5 Gew.-% | **T6,** 5 Gew.-% |
| **F20** *) | **E2,** 5 Gew.-% | **T7,** 5 Gew.-% |

| | | |
|---|---|---|
| *) erfindungsgemäß **) nicht erfindungsgemäß | | |

### Beispiel 12: Anwendungsbeispiele:

Zur Beurteilung der gewünschten Effekte bzgl. Weichgriff, Tropfeneinsinkzeit, Easy Ironing und Knittererholungswinkel wurden alle ausgerüsteten Textilien zusammen mit ca. 2 kg Ballaststoff in einer Haushaltswaschmaschine der Marke MIELE Softronic W 1935 WPS EcoLine mit dem Koch-/Buntwäscheprogramm bei 40°C gewaschen und bei 1200 UpM geschleudert. Als Waschtensid wurden dabei 65 g eines Waschpulvers Testwaschmittel ECE-2 der Firma WFK dosiert. Nach dem Waschgang wird die Formulierung **F1** bis **F8** (vorverdünnt in 1 Liter Leitungswasser mit 16°dH) über das Spülfach hinzugefügt. Anschließend wurden zwei weitere Waschzyklen von jeweils 90 Minuten Dauer ohne Zwischentrocknung durchgeführt. Abschließend wurde die Ware mindestens 12 Stunden im Klimaraum bei 23°C und 60% Luftfeuchtigkeit auf der Leine getrocknet.

### Bestimmung des Weichgriffs (Griffbewertung):

Da der Weichgriff von Textilien stark dem subjektiven Empfinden der Testpersonen unterliegt, kann nur eine Standardisierung der Randbedingungen, nicht aber der Bewertung erreicht werden. Um trotzdem eine Reproduzierbarkeit zu gewährleisten, wurden die ausgerüsteten Muster hinsichtlich ihres Weichgriffs beurteilt und in eine Rangfolge gebracht. Dazu wurden von 10 Personen in Abhängigkeit der Anzahl n der getesteten Muster 1 bis n Punkte vergeben, wobei n Punkte für das weichste Muster und 1 Punkt für das am wenigsten weich ausgerüstete Muster vergeben wurden. Das unausgerüstete Referenzmuster erhielt 0 Punkte. Die Griffbewertung eines Musters errechnet sich somit als Mittelwert der jeweils auf dieses Muster entfallenen Punkte.

Die Ergebnisse zum Weichgriff für die Formulierungen **F1** bis **F8** sind in Tabelle 3 zusammengefasst.

**Tabelle 3: Bestimmung des Weichgriffs**

| Weichgriffbeurteilung auf Frottierware(Flächengewicht 500 g/m²) nach Waschmaschinenbehandlung und Trocknung | |
|---|---|
| **F1** (erfindungsgemäß) | +++ |
| **F2** (erfindungsgemäß) | +++ |
| **F3** (erfindungsgemäß) | ++ |
| **F4** (nicht erfindungsgemäß) | + |
| **F5** (nicht erfindungsgemäß) | ○ |
| **F6** (nicht erfindungsgemäß) | ○ |
| **F7** (erfindungsgemäß) | +++ |
| **F8** (erfindungsgemäß) | +++ |
| +++ exzellent weich, ++ sehr weich, + weich, o hart | |

Die Ausrüstung des Textils mit Formulierung **F4** weist einen weichen Griff auf, das Textil wirkt an der Oberfläche schmierig.

Die Ausrüstung des Textils mit Formulierungen **F5** bzw. **F6** weist in der Applikation in der Waschmaschine überraschenderweise einen harten Griff auf.

Die Ausrüstung des Textils mit Formulierungen **F1** bis **F3** sowie **F7** oder **F8** führt zu einem natürlichen, angenehmen Weichgriff, der sich insbesondere im Falle von Formulierung **F2** oder **F8** trocken, voluminös in keinem Falle schmierig an der Oberfläche anfühlt.

Die Ausrüstung des Textils mit einer Kombination aus Carbamato-funktionalisiertem Polydiorganosiloxane und kationischem Tensid führt überraschenderweise zu einem eindeutig besseren Weichgriff als die Ausrüstung des Textils mit einem kationischen Tensid oder vor allem dem Carbamato-funktionalisierten Polydiorganosiloxan allein.

Bei der Ausrüstung mit den erfindungsgemäßen Formulierungen wurde eine Vergilbung der Textilien nicht festgestellt, was insbesondere bei den Formulierungen, die Emulsionen aus Carbamato-funktionalisiertem Polydimethylsiloxan **2** mit niedrigem Funktionalisierungsgrad der Amino-Gruppen enthalten, überraschend ist.

In analoger Weise wurde der Weichgriff der erfindungsgemäßen Formulierungen **F15** bis **F20** mit dem Weichgriff der nicht erfindungsgemäßen Formulierungen **F9** bis **F14** verglichen. Es war ebenfalls eine Verbesserung des Weichgriffs mit den erfindungsgemäßen Formulierungen **F15** bis **F20** festzustellen.

### Tropfeneinsinkzeit:

Auf das ausgerüstete Textil wurde nach der Waschmaschinenbehandlung und Trocknung ein Tropfen entionisiertes Wasser aus einer Höhe von 4 cm auf die gespannte Stoffoberfläche gegeben und die Zeit bestimmt, nach der der Wassertropfen vom Stoff aufgesaugt war. Es wurden fünf Bestimmungen durchgeführt und der Mittelwert gebildet.

Die Ergebnisse zur Tropfeneinsinkzeit für die erfindungsgemäßen Formulierungen im Vergleich zur nicht-erfindungsgemäßen Formulierung sind in Tabelle 4 zusammengefasst.

**Tabelle 4: Bestimmung der Tropfeneinsinkzeit**

| Tropfeneinsinkzeit auf Frottierware(Flächengewicht 500 g/m²) nach Waschmaschinenbehandlung und Trocknung | |
|---|---|
| **F1** (erfindungsgemäß) | +++ |
| **F2** (erfindungsgemäß) | +++ |
| **F4** (nicht erfindungsgemäß) | + |
| +++ 0-3 Sekunden, ++ 3-5 Sekunden, + 5-25 Sekunden | |

Die Ausrüstung des Textils mit einer Kombination aus Carbamato-funktionalisiertem Polydiorganosiloxane und kationischem Tensid (**F1** und **F2,** erfindungsgemäß) führt zu einer deutlich verringerten Tropfeneinsinkzeit auf Baumwollwirkware als die Ausrüstung mit dem kationischen Tensid (**F4,** nicht erfindungsgemäß) allein. Dadurch erreicht man bei dem Textil sowohl eine verbesserte Weichheit wie auch eine bessere Wasseraufnahme als bei der Ausrüstung des Textils mit Formulierungen gemäß dem Stand der Technik.

### Easy Ironing

Das ausgerüstete und (wie oben angegebene) gewaschene und getrocknete Textil wird auf eine schiefe Ebene gespannt. Die schiefe Ebene hat eine Länge von 100 cm und einen Neigungswinkel von 13,5°. Ein Handelsübliches Bügeleisen der Marke Tefal wird erhitzt (Stellung Cotton), auf das obere Ende der Ebene gesetzt und die schiefe Ebene herabgleiten gelassen. Die Zeit bis zum Erreichen des unteren Endes der schiefen Ebene wird gemessen. Diese Prozedur wird wiederholt bis ein konstanter Wert erreicht wird. Die Hitze des Bügeleisens wird vor jeder Prozedur erneut eingestellt.

Die Ergebnisse zum Easy Ironing für die erfindungsgemäßen Formulierungen im Vergleich zur nicht-erfindungsgemäßen Formulierung sind in Tabelle 5 zusammengefasst.

**Tabelle 5: Bestimmung des Easy Ironing**

| Easy Ironing auf 100% Baumwolle (Flächengewicht 170 g/m²) nach Waschmaschinenbehandlung und Trocknung | |
|---|---|
| **F1** (erfindungsgemäß) | 0,75 m/Sek. |
| **F2** (erfindungsgemäß) | 0,41 m/Sek |
| **F4** (nicht erfindungsgemäß) | 0,05 m/Sek. |

Die Ausrüstung des Textils mit Formulierung **F1** oder **F2** führt im Vergleich zur Formulierung **F4** zu einer deutlich höheren Gleitgeschwindigkeit, somit einem deutlich reduzierten Kraftaufwand, der zum Bügeln des Textils notwendig ist.

### Knittererholungswinkel

Die Bestimmung erfolgt nach der Methode DIN 53 890/1972 (dort ist auch die exakte Beschreibung der Methode zu entnehmen):
Aus dem zu prüfenden Textil werden Proben der Größe 50 mm Länge und 20 mm Breite entnommen. Jede Probe wird in Querrichtung umgefaltet, so dass die Länge des umzulegenden Probenschenkels 10 mm beträgt. Unter den umzulegenden Probenschenkel wird eine Metallfolie von 0,15 mm Dicke gelegt, um ein Anhaften der Fasern zu verhindern. Die Probe wird mit einem Objektträger bedeckt und einem Gewichtstück von 1000 g so belastet, das die Belastung nur auf dem umgelegten Probenschenkel ruht. Die Belastungsdauer beträgt 30 min.

Nach dem Abnehmen des Gewichtsstückes des Objektträgers wird der allmählich größer werdende Knittererholungswinkel nach Verlauf von 5 und 30 min an beiden Seiten des Winkelschenkels mit einem Winkelmesser bestimmt.

Pro Textil sind mindestens je 10 Proben vorzubereiten und zu vermessen. Die angegebenen Messergebnisse sind die Mittelwerte der jeweiligen Bestimmungen.

Die Ergebnisse zum Knittererholungswinkel auf Baumwolle bzw. auf Mischgewebe für die erfindungsgemäßen Formulierungen im Vergleich zur nicht-erfindungsgemäßen Formulierung sind in den Tabellen 6 und 7 zusammengefasst.

**Tabelle 6: Bestimmung des Knittererholungswinkels auf Baumwolle**

| Knittererholungswinkel auf 100% Baumwolle (Flächengewicht 170 g/m²) nach Waschmaschinenbehandlung und Trocknung | | |
|---|---|---|
| | nach 5 min | nach 30 min |
| **F1** (erfindungsgemäß) | 41° | 45° |
| **F2** (erfindungsgemäß) | 37° | 43° |
| **F4** (nicht erfindungsgemäß) | 21° | 33° |

**Tabelle 7: Bestimmung des Knittererholungswinkels auf Mischgewebe**

| Knittererholungswinkel auf Mischgewebe (Flächengewicht: 125 g/m²) nach Waschmaschinenbehandlung und Trocknung | | |
|---|---|---|
| | nach 5 min | nach 30 min |
| **F2** (erfindungsgemäß) | 97° | 108° |
| **F4** (nicht erfindungsgemäß) | 69° | 77° |

Die Ausrüstung des Textils mit einer Kombination aus Carbamato-funktionalisiertem Polydiorganosiloxane und kationischem Tensid (**F1** und **F2,** erfindungsgemäß) führt zu einem deutlich erhöhten Knittererholungswinkel bei Baumwollwirkware und bei Mischgewebeware als die Ausrüstung mit dem kationischen Tensid (**F4,** nicht erfindungsgemäß) allein. Dadurch erreicht man bei dem Textil eine deutlich verringerte Knitterneigung als dies der Stand der Technik liefert.

## Patentansprüche

1. Zusammensetzungen, enthaltend
(A) mindestens 0,1 Gew.-% und höchstens 10,0 Gew.-% Carbamato-funktionalisierte Organopolysiloxane, die durchschnittlich je Molekül mindestens eine Carbamato-funktionelle Gruppe Y der Formel
-R⁴-[NX-R⁵-]ₙNX-H
enthalten, wobei
X gleich oder verschieden ist und ein Wasserstoffatom oder einen Rest Z der Formel
-CO-O-CHR⁶-CH₂-OH oder -CO-O-CH₂-CHR⁶-OH
bedeutet, wobei durchschnittlich je Molekül mindestens ein Rest X ein Rest Z ist,
wobei R⁴ gleich oder verschieden ist und einen zweiwertigen, Si-C-gebundenen Kohlenwasserstoffrest mit 1 bis 18 C-Atomen bedeutet,
R⁵ gleich oder unterschiedlich ist und einen zweiwertigen Kohlenwasserstoffrest mit 1 bis 6 C-Atomen bedeutet,
R⁶ gleich oder unterschiedlich ist und ein Wasserstoffatom oder einen einwertigen Kohlenwasserstoffrest mit 1 bis 36 C-Atomen bedeutet, wobei eine oder mehrere -CH₂-Gruppen durch Heteroatome, vorzugsweise durch -O- oder -S-, ersetzt sein können, n gleich 1, 2, 3 oder 4 ist,
(B) mindestens 1 Gew.-% und höchstens 40 Gew.-% kationische Tenside
und
(C) mindestens 30 Gew.-% und höchstens 97 Gew.-% Wasser, jeweils bezogen auf das Gesamtgewicht der wässrigen Zusammensetzungen.

2. Zusammensetzungen nach Anspruch 1, **dadurch gekennzeichnet, dass** als Carbamato-funktionalisierte Organopolysiloxane (A) Carbamato-funktionalisierte Polydiorganosiloxane der Formel
[R¹₂R²SiO_{1/2}]₂[R²(Y)SiO_{2/2}]ₖ[R¹₂SiO_{2/2}]ₘ (I),
wobei
R¹ gleich oder verschieden ist und einen einwertigen Si-C-gebundenen Kohlenwasserstoffrest mit 1 bis 18 C-Atomen bedeutet,
R² gleich oder unterschiedlich ist und gleich Rest R¹ oder eine Hydroxygruppe -OH oder eine Alkoxy-Gruppe der Formel -O-R³ bedeutet, wobei R³ ein gegebenenfalls substituierter Alkylrest mit 1-8 C-Atomen ist,
Y die im Anspruch 1 dafür angegebene Bedeutung hat,
wobei durchschnittlich je Molekül mindestens eine Gruppe Y einen Rest Z der Formel
-CO-O-CHR⁶-CH₂-OH
oder
-CO-O-CH₂-CHR⁶-OH
enthält, wobei R⁶ die im Anspruch 1 dafür angegebene Bedeutung hat,
m eine ganze Zahl und mindestens 40 und höchstens 1000 ist
k eine ganze Zahl und mindestens 1 und höchstens 40 ist,
wobei das Verhältnis von m zu k mindestens 25 und höchstens 1000 ist,
eingesetzt werden.

3. Zusammensetzungen nach Anspruch 1 oder 2, **dadurch gekennzeichnet, dass** bei den Carbamato-funktionalisierten Organopolysiloxanen (A) mindestens 5 Mol% und weniger als 50 Mol% der N-gebundenen Reste X in den Gruppen Y nicht ein Wasserstoffatom sind, sondern die Bedeutung des Restes Z der Formel
-CO-O-CHR⁶-CH₂-OH
oder
-CO-O-CH₂-CHR⁶-OH
haben, wobei R⁶ die im Anspruch 1 dafür angegebene Bedeutung hat.

4. Zusammensetzungen nach Anspruch 1, 2 oder 3, **dadurch gekennzeichnet, dass** R⁶ ein Methylrest ist.

5. Zusammensetzungen nach einem der Ansprüche 1 bis 4, **dadurch gekennzeichnet, dass** die Carbamato-funktionalisierten Organopolysiloxane (A) in Form ihrer wässrigen Emulsionen enthaltend
Carbamato-funktionalisierte Organopolysiloxane (A) nach einem der Ansprüche 1 bis 4,
nicht-ionische und/oder kationische Emulgatoren und Wasser,
eingesetzt werden.

6. Zusammensetzungen nach einem der Ansprüche 1 bis 5, **dadurch gekennzeichnet, dass** als kationische Tenside
(B1) Quartäre Ammonium-Tenside der Formel 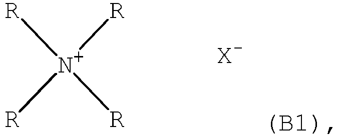
wobei R unabhängig voneinander ein Wasserstoffatom, eine Alkylgruppe, eine Hydroxyalkylgruppe, eine Benzylgruppe oder eine Gruppe der Formel -(CₚH₂ₚO)_{q}-H ist, wobei p eine ganze Zahl von 1 bis 4 und q eine ganze Zahl von 2 bis 5 ist, und
X⁻ ein Halogenid-, ein Sulfat-, ein Sulfonat-, ein Nitrat-, ein Methylsulfat- oder ein Ethylsulfat-Anion,
oder
(B2) Ester-/Amido-haltige quartäre Ammonium-Tenside der Formel 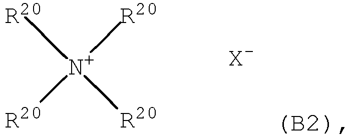
wobei mindestens ein Rest R²⁰ ein einwertiger Kohlenwasserstoffrest ist, der eine Gruppe G der Formel -O-(O)C-, -C(O)-O-, -NR-C(O)-, oder -C(O)-NR- oder -CR=NR enthält,
und die weiteren Resten R²⁰ unabhängig voneinander eine Gruppe R sind
oder ein Rest der Formel -R²⁰'-N⁺R²⁰₃ X⁻ sind,
wobei R^{20'} eine Alkylen-Gruppe ist,
die zwei Ammonium-Gruppen R²⁰₃N⁺ X⁻ miteinander zu einer Verbindung R²⁰₃N-Alkylen-NR²⁰₃ 2X⁻ verknüpft,
oder zwei Reste R²⁰ einen gegebenenfalls ungesättigten Ring formen, und
R und X⁻ die oben dafür angegebene Bedeutung haben,
eingesetzt werden.

7. Zusammensetzungen nach Anspruch 6, **dadurch gekennzeichnet, dass** als Ester-/Amido-haltige quartäre Ammonium-Tenside (B2) solche ausgewählt aus der Gruppe von
(B2-1) Tensiden der Formel
R²⁵₍₄₋ᵣ₎(R²⁶-G¹-R²⁷)ᵣN⁺X⁻ (B2-1),
wobei
R²⁵ unabhängig voneinander ein Wasserstoffatom, eine Alkylgruppe mit 1 bis 3 C-Atomen, eine Hydroxyalkylgruppe mit 1 bis 3 C-Atomen, eine Benzylgruppe oder eine Gruppe der Formel -(CₚH₂ₚO)_{q}-H bedeutet, wobei p eine ganze Zahl von 1 bis 4, bevorzugt 2 ist, und q eine ganze Zahl von 2 bis 5 ist, R²⁶ ein einwertiger Kohlenwasserstoffrest, bevorzugt ein Alkyl- oder Alkenylrest mit 12 bis 24 C-Atomen, ist, R²⁷ ein zweiwertiger, linearer oder verzweigter Alkylenrest mit 1 bis 6 C-Atomen ist,
G¹ eine Gruppe -O-(O)C-, -C(O)-O-, -NR-C(O)-,
oder -C(0)NR- ist,
r 2 oder 3 ist und
X⁻ ein Halogenid-, ein Sulfat-, ein Sulfonat-, ein Nitrat-, ein Methylsulfat- oder ein Ethylsulfat-Anion ist,
(B2-2) Tensiden der Formel 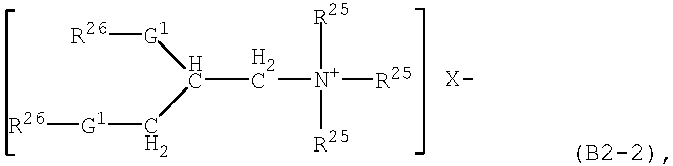 wobei
R²⁵, R²⁶, G¹ und X⁻ die oben dafür angegebene Bedeutung haben,
(B2-3) Tensiden der Formel 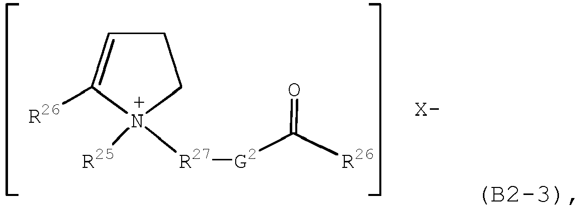 wobei
G² ein Sauerstoff-Atom oder die Gruppe NR²⁵ ist, R²⁵, R²⁶, R²⁷ und X⁻ die oben dafür angegebene Bedeutung haben,
(B2-4) Tensiden der Formel 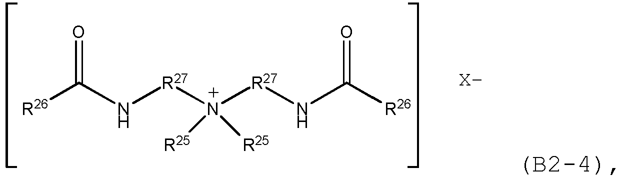 wobei
R²⁵, R²⁶, R²⁷ und X⁻ die oben dafür angegebene Bedeutung haben,
(B2-5) Tensiden der Formel 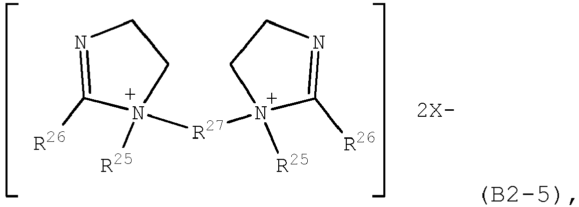 wobei
R²⁵, R²⁶, R²⁷ und X⁻ die oben dafür angegebene Bedeutung haben,
und deren Mischungen
eingesetzt werden.

8. Zusammensetzungen nach einem der Ansprüche 1 bis 5, **dadurch gekennzeichnet, dass** die Carbamato-funktionalisierten Organopolysiloxane (A) hergestellt werden durch Umsetzung von Amino-funktionalisierten Organopolysiloxanen (A'), die durchschnittlich je Molekül mindestens eine Gruppe Y' der Formel
-R⁴- [NH-R⁵-]ₙNH₂
enthalten, wobei R⁴, R⁵ und n die in Anspruch 1 dafür angegebene Bedeutung haben, mit cyclischen Carbonaten der Formel 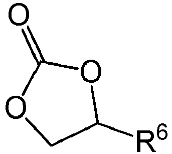 wobei
R⁶ die in Anspruch 1 oder 4 dafür angegebene Bedeutung hat.

9. Zusammensetzungen nach Anspruch 8, **dadurch gekennzeichnet, dass** als Amino-funktionalisierte Organopolysiloxane (A') Amino-funktionalisierte Polydiorganosiloxane (A') der Formel
[R¹₂R²SiO_{1/2}]₂[R²(Y')SiO_{2/2}]ₖ[R¹₂SiO_{2/2}]ₘ (I'),
wobei
R¹, R², m und k die im Anspruch 2 dafür angegebene Bedeutung haben und
Y' die im Anspruch 8 dafür angegebene Bedeutung hat, eingesetzt werden.

10. Zusammensetzungen nach Anspruch 8 oder 9, **dadurch gekennzeichnet, dass** als Amino-funktionalisierte Organopolysiloxane (A') solche eingesetzt werden, die eine Aminzahl von höchstens 0,4 mmol/g aufweisen.

11. Wasch- und Reinigungsmittel enthaltend Zusammensetzungen nach einem der Ansprüche 1 bis 7.

12. Verfahren zur Pflege und Reinigung von Fasern, insbesondere Textilfasern und Textilgeweben, mit den Zusammensetzungen nach einem der Ansprüche 1 bis 7 oder mit den Wasch- und Reinigungsmitteln nach Anspruch 11 enthaltend diese Zusammensetzungen.

13. Kosmetische Formulierungen, insbesondere Haarbehandlungsmittel, enthaltend Zusammensetzungen nach einem der Ansprüche 1 bis 7.

14. Verfahren zur Pflege und Reinigung von Haaren mit den Zusammensetzungen nach einem der Ansprüche 1 bis 7 oder mit den kosmetischen Formulierungen nach Anspruch 13 enthaltend diese Zusammensetzungen.

## Claims

1. Compositions comprising
(A) at least 0.1% by weight and at most 10.0% by weight of carbamato-functionalized organopolysiloxanes containing an average per molecule of at least one carbamato-functional Y group of the formula
-R⁴-[NX-R⁵-]ₙNX-H
where
X is the same or different and is a hydrogen atom or a Z radical of the formula
-CO-O-CHR⁶-CH₂-OH
or
-CO-O-CH₂-CHR⁶-OH
where an average of at least one X radical per molecule is a Z radical,
where R⁴ is the same or different and is a divalent, Si-C-bonded hydrocarbyl radical having 1 to 18 carbon atoms,
R⁵ is the same or different and is a divalent hydrocarbyl radical having 1 to 6 carbon atoms, R⁶ is the same or different and is a hydrogen atom or a monovalent hydrocarbyl radical having 1 to 36 carbon atoms, where one or more -CH₂-groups may be replaced by heteroatoms, preferably by -O- or -S-,
n is 1, 2, 3 or 4,
(B) at least 1% by weight and at most 40% by weight of cationic surfactants
and
(C) at least 30% by weight and at most 97% by weight of water,
based in each case on the total weight of the aqueous compositions.

2. Compositions according to Claim 1, **characterized in that** the carbamato-functionalized organopolysiloxanes (A) used are carbamato-functionalized polydiorganosiloxanes of the formula
[R¹₂R²SiO_{1/2}]₂[R²(Y)SiO_{2/2}]ₖ[R¹₂SiO_{2/2}]ₘ (I)
where
R¹ is the same or different and is a monovalent Si-C-bonded hydrocarbyl radical having 1 to 18 carbon atoms,
R² is the same or different and is a R¹ radical or a hydroxyl group -OH or alkoxy group of the formula -O-R³ where R³ is an optionally substituted alkyl radical having 1-8 carbon atoms,
Y is as defined in Claim 1,
where an average of at least one Y group per molecule contains a Z radical of the formula
-CO-O-CHR⁶-CH₂-OH
or
-CO-O-CH₂-CHR⁶-OH
where R⁶ is as defined in Claim 1,
m is an integer and is at least 40 and at most 1000,
k is an integer and is at least 1 and at most 40,
where the ratio of m to k is at least 25 and at most 1000.

3. Compositions according to Claim 1 or 2, **characterized in that**, in the carbamato-functionalized organopolysiloxanes (A), at least 5 mol% and less than 50 mol% of the N-bonded X radicals in the Y groups are not a hydrogen atom, but are defined as the Z radical of the formula
-CO-O-CHR⁶-CH₂-OH
or
-CO-O-CH₂-CHR⁶-OH
where R⁶ is as defined in Claim 1.

4. Compositions according to Claim 1, 2 or 3, **characterized in that** R⁶ is a methyl radical.

5. Compositions according to any of Claims 1 to 4, **characterized in that** the carbamato-functionalized organopolysiloxanes (A) are used in the form of aqueous emulsions thereof comprising carbamato-functionalized organopolysiloxanes (A) according to any of Claims 1 to 4,
nonionic and/or cationic emulsifiers and
water.

6. Compositions according to any of Claims 1 to 5, **characterized in that** the cationic surfactants used are
(B1)quaternary ammonium surfactants of the formula 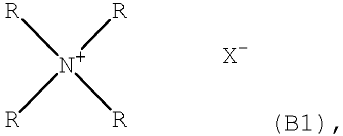
where R is independently a hydrogen atom, an alkyl group, a hydroxyalkyl group, a benzyl group or a group of the formula -(CₚH₂ₚO)_{q}-H where p is an integer from 1 to 4 and q is an integer from 2 to 5, and
X⁻ is a halide, a sulfate, a sulfonate, a nitrate, a methylsulfate or an ethylsulfate anion,
or
(B2)ester/amido-containing quaternary ammonium surfactants of the formula 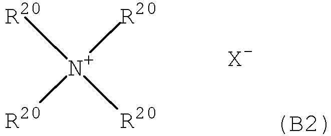
where at least one R²⁰ radical is a monovalent hydrocarbyl radical containing a G group of the formula
-O-(O)C-, -C(O)-O-, -NR-C(O)-, or -C(O)-NR- or -CR=NR,
and the further R²⁰ radicals are independently an R group
or a radical of the formula -R^{20'}-N⁺R²⁰₃X⁻ where R^{20'} is an alkylene group,
the two ammonium groups R²⁰₃N⁺ X⁻ are joined to one another to form an R²⁰₃N-alkylene-NR²⁰₃ 2X⁻ compound,
or two R²⁰ radicals form an optionally unsaturated ring, and
R and X⁻ are as defined above.

7. Compositions according to Claim 6, **characterized in that** the ester/amido-containing quaternary ammonium surfactants (B2) used are those selected from the group of
(B2-1)surfactants of the formula
R²⁵₍₄₋ᵣ₎(R²⁶-G¹-R²⁷)ᵣN⁺X⁻ (B2-1)
where
R²⁵ is independently a hydrogen atom, an alkyl group having 1 to 3 carbon atoms, a hydroxyalkyl group having 1 to 3 carbon atoms, a benzyl group or a group of the formula -(CₚH₂ₚO)_{q}-H where p is an integer from 1 to 4, preferably 2, and q is an integer from 2 to 5,
R²⁶ is a monovalent hydrocarbyl radical, preferably an alkyl or alkenyl radical having 12 to 24 carbon atoms,
R²⁷ is a divalent linear or branched alkylene radical having 1 to 6 carbon atoms,
G¹ is a -O-(O)C-, -C(O)-O- -NR-C(O)-, or -C(O)NR-group,
r is 2 or 3 and
X⁻ is a halide, a sulfate, a sulfonate, a nitrate, a methylsulfate or an ethylsulfate anion,
(B2-2) surfactants of the formula 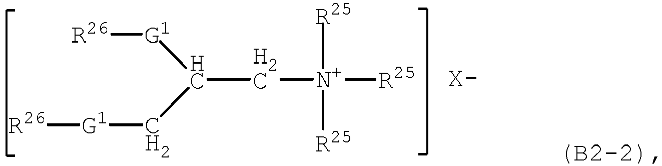 where
R²⁵, R²⁶, G¹ and X⁻ are as defined above,
(B2-3) surfactants of the formula 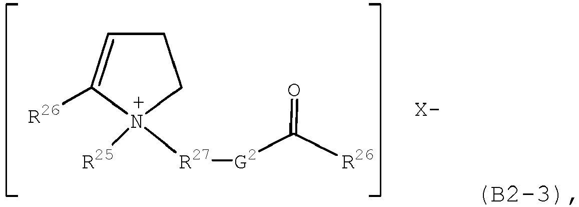 where
G² is an oxygen atom or the NR²⁵ group,
R²⁵, R²⁶, R²⁷ and X⁻ are as defined above,
(B2-4) surfactants of the formula 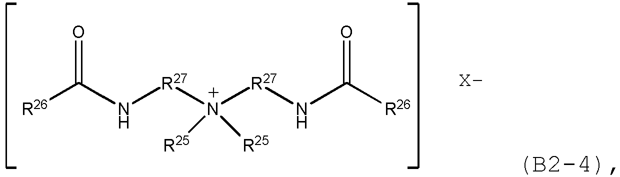 where
R²⁵, R²⁶, R²⁷ and X⁻ are as defined above,
(B2-5) surfactants of the formula 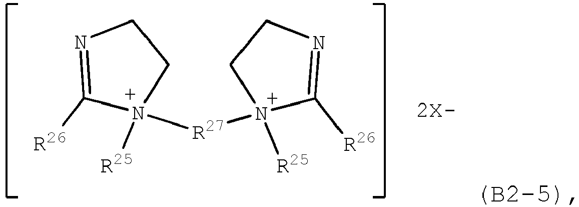 where
R²⁵, R²⁶, R²⁷ and X⁻ are as defined above,
and mixtures thereof.

8. Compositions according to any of Claims 1 to 5, **characterized in that** the carbamato-functionalized organopolysiloxanes (A) are prepared by reaction of amino-functionalized organopolysiloxanes (A') containing an average per molecule of at least one Y' group of the formula
-R⁴-[NH-R⁵-]ₙNH₂
where R⁴, R⁵ and n are as defined in Claim 1 with cyclic carbonates of the formula 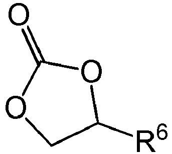 where
R⁶ is as defined in Claim 1 or 4.

9. Compositions according to Claim 8, **characterized in that** the amino-functionalized organopolysiloxanes (A') used are amino-functionalized polydiorganosiloxanes (A') of the formula
[R¹₂R²SiO_{1/2}]₂[R²(Y')SiO_{2/2}]ₖ[R¹₂SiO_{2/2}]ₘ (I')
where
R¹, R², m and k are as defined in Claim 2 and
Y' is as defined in Claim 8.

10. Compositions according to Claim 8 or 9, **characterized in that** the amino-functionalized organopolysiloxanes (A') used are those having an amine value of at most 0.4 mmol/g.

11. Washing and cleaning compositions comprising compositions according to any of Claims 1 to 7.

12. Method of fiber care and cleaning, especially of textile fibers and textile fabrics, with the compositions according to any of Claims 1 to 7 or with the washing and cleaning compositions according to Claim 11 comprising these compositions.

13. Cosmetic formulations, especially hair treatment compositions, comprising compositions according to any of Claims 1 to 7.

14. Method of hair care and washing with the compositions according to any of Claims 1 to 7 or with the cosmetic formulations according to Claim 13 comprising these compositions.

## Revendications

1. Compositions, contenant
(A) Au moins 0,1% en poids et au maximum 10,0% en poids d'organopolysiloxanes fonctionnalisés par carbamato, qui contiennent en moyenne, par molécule, au moins un groupe fonctionnel Y de type carbamato de formule
-R⁴-[NX-R⁵-]ₙNX-H
X étant identique ou différent et signifiant un atome d'hydrogène ou un radical Z de formule -CO-O-CHR⁶-CH₂-OH ou -CO-O-CH₂-CHR⁶-OH, en moyenne, par molécule, au moins un radical X étant un radical Z,
R⁴ étant identique ou différent et signifiant un radical hydrocarboné lié par Si-C, divalent, comportant 1 à 18 atome(s) de C,
R⁵ étant identique ou différent et signifiant un radical hydrocarboné divalent comportant 1 à 6 atome(s) de C,
R⁶ étant identique ou différent et signifiant un atome d'hydrogène ou un radical hydrocarboné monovalent comportant 1 à 36 atome(s) de C, un ou plusieurs groupes -CH₂ pouvant être remplacés par des hétéroatomes, de préférence par -O- ou -S-,
n étant égal à 1, 2, 3 ou 4,
(B) au moins 1 % en poids et au plus 40 % en poids de tensioactifs cationiques
et
(C) au moins 30 % en poids et au plus 97 % en poids d'eau, à chaque fois par rapport au poids total des compositions aqueuses.

2. Compositions selon la revendication 1, **caractérisées en ce que** des polydiorganosiloxanes fonctionnalisés par carbamato de formule
[R¹₂R²SiO_{1/2}]₂[R²(Y)SiO_{2/2}]ₖ[R¹₂SiO_{2/2}]ₘ (I).
R¹ étant identique ou différent et signifiant un radical hydrocarboné monovalent lié par Si-C comportant 1 à 18 atome(s) de C,
R² étant identique ou différent et étant égal au radical R¹ ou signifiant un groupe hydroxy -OH ou un groupe alcoxy de formule -O-R³, R³ étant un radical alkyle éventuellement substitué comportant 1 à 8 atome(s) de C,
Y possédant la signification indiquée pour celui-ci dans la revendication 1,
au moins un groupe Y contenant en moyenne par molécule un radical Z de formule -CO-O-CHR⁶-CH₂-OH ou -CO-O-CH₂-CHR⁶-OH, R⁶ possédant la signification indiquée pour celui-ci dans la revendication 1,
m étant un nombre entier et étant au moins 40 et au plus 1000
k étant un nombre entier et étant au moins 1 et au plus 40,
le rapport de m à k étant d'au moins 25 et d'au plus 1000,
sont utilisés en tant qu'organopolysiloxanes (A) fonctionnalisés par carbamato.

3. Compositions selon la revendication 1 ou 2, **caractérisées en ce que** dans les organopolysiloxanes (A) fonctionnalisés par carbamato, au moins 5 % en moles et moins de 50 % en moles des radicaux X N-liés dans les groupes Y ne sont pas un atome d'hydrogène, mais possèdent la signification du radical Z de formule
-CO-O-CHR⁶-CH₂-OH ou -CO-O-CH₂-CHR⁶-OH, R⁶ possédant la signification indiquée pour celui-ci dans la revendication 1.

4. Compositions selon la revendication 1, 2 ou 3, **caractérisées en ce que** R⁶ est un radical méthyle.

5. Compositions selon l'une quelconque des revendications 1 à 4, **caractérisées en ce que** les organopolysiloxane (A) fonctionnalisés par carbamato sont utilisés sous forme de leurs émulsions aqueuses contenant
des organopolysiloxane (A) fonctionnalisés par carbamato selon l'une quelconque des revendications 1 à 4,
des émulsifiants non ioniques et/ou cationiques et de l'eau.

6. Compositions selon l'une quelconque des revendications 1 à 5, **caractérisées en ce que**
(B1) des tensioactifs de type ammonium quaternaire de formule 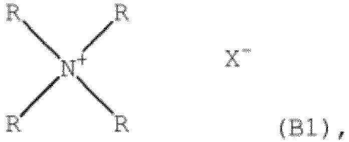
R étant indépendamment les uns des autres un atome d'hydrogène, un groupe alkyle, un groupe hydroxyalkyle, un groupe benzyle ou un groupe de formule -(CₚH₂ₚO)_{q}-H, p étant un nombre entier de 1 à 4 et q étant un nombre entier de 2 à 5, et
X⁻ étant un anion halogénure, un anion sulfate, un anion sulfonate, un anion nitrate, un anion méthylsulfate ou un anion éthylsulfate,
ou
(B2) des tensioactifs de type ammonium quaternaire contenant ester/amido de formule 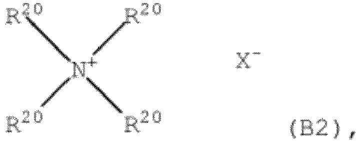
au moins un radical R²⁰ étant un radical hydrocarboné monovalent, qui contient un groupe G de formule -O-(O)C-, -C(O)-O-, -NR-C(O)-, ou -C(O)-NR- ou -CR=NR, et les autres radicaux R²⁰ étant indépendamment les uns des autres un groupe R
ou sont un radical de formule -R^{20'}-N⁺R²⁰₃X⁻,
R^{20'} étant un groupe alkylène,
qui relie ensemble deux groupes ammonium R²⁰₃N⁺X⁻ en un composé R²⁰₃N-alkylène-NR²⁰₃2X⁻,
ou deux radicaux R²⁰ formant un cycle éventuellement insaturé, et
R et X⁻ possédant la signification indiquée ci-dessus pour ceux-ci,
sont utilisés en tant que tensioactifs cationiques.

7. Compositions selon la revendication 6, **caractérisées en ce qu'**en tant que tensioactifs (B2) de type ammonium quaternaire contenant ester/amido, ceux choisis dans le groupe
(B2-1) des tensioactifs de formule
R²⁵₍₄₋ᵣ₎(R²⁶-G¹-R²⁷)ᵣN⁺X⁻ (B2-1),
R²⁵ signifiant indépendamment les uns des autres un atome d'hydrogène, un groupe alkyle comportant 1 à 3 atome(s) de C, un groupe hydroxyalkyle comportant 1 à 3 atome(s) de C, un groupe benzyle ou un groupe de formule -(CₚH₂ₚO)_{q}-H, p étant un nombre entier de 1 à 4, préférablement étant 2, et q étant un nombre entier de 2 à 5,
R²⁶ étant un radical hydrocarboné monovalent, préférablement un radical alkyle ou alcényle comportant 12 à 24 atomes de C,
R²⁷ étant un radical alkylène divalent, linéaire ou ramifié comportant 1 à 6 atome(s) de C,
G¹ étant un groupe -O-(O)C-, -C(O)-O-, -NR-C(O)-, ou -C(O)NR-,
r étant 2 ou 3 et
X⁻ étant un anion halogénure, un anion sulfate, un anion sulfonate, un anion nitrate, un anion méthylsulfate ou un anion éthylsulfate,
(B2-2) des tensioactifs de formule 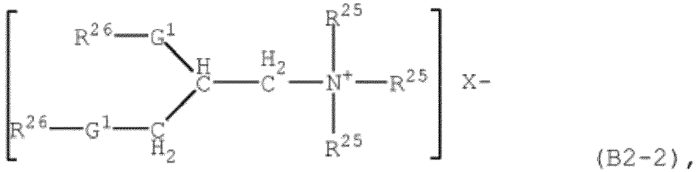 R²⁵, R²⁶, G¹ et X⁻ possédant la signification indiquée ci-dessus pour ceux-ci,
(B2-3) des tensioactifs de formule 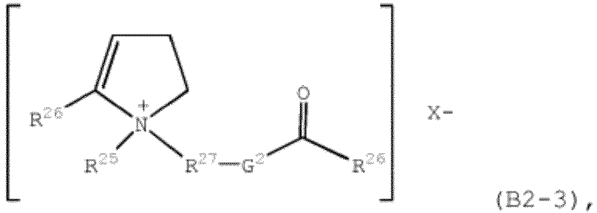
G² étant un atome d'oxygène ou le groupe NR²⁵,
R²⁵, R²⁶, R²⁷ et X⁻ possédant la signification indiquée ci-dessus pour ceux-ci,
(B2-4) des tensioactifs de formule 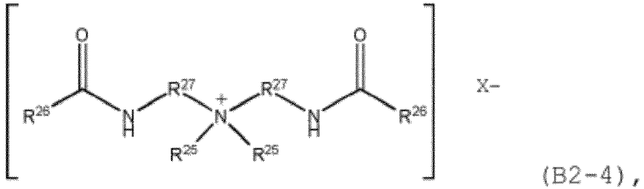 R²⁵, R²⁶, R²⁷ et X⁻ possédant la signification indiquée ci-dessus pour ceux-ci,
(B2-5) des tensioactifs de formule 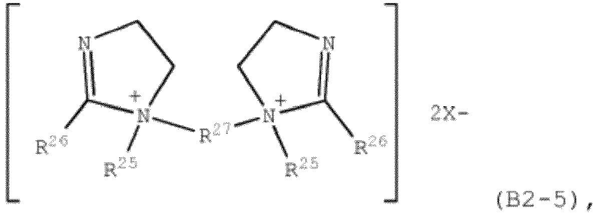 R²⁵, R²⁶, R²⁷ et X⁻ possédant la signification indiquée ci-dessus pour ceux-ci,
et leurs mélanges
sont utilisés.

8. Compositions selon l'une quelconque des revendications 1 à 5, **caractérisées en ce que** les organopolysiloxanes (A) fonctionnalisés par carbamato sont préparés par transformation d'organopolysiloxanes (A') fonctionnalisés par amino, qui contiennent en moyenne par molécule au moins un groupe Y' de formule
-R⁴-[NH-R⁵-]ₙNH₂,
R⁴, R⁵ et n possédant la signification indiquée pour ceux-ci dans la revendication 1, avec des carbonates cycliques de formule 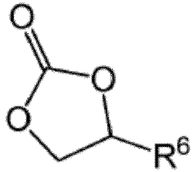 R⁶ possédant la signification indiquée pour celui-ci dans la revendication 1 ou 4.

9. Compositions selon la revendication 8, **caractérisées en ce qu'**en tant qu'organopolysiloxanes (A') fonctionnalisés par amino, des polydiorganosiloxanes (A') fonctionnalisés par amino de formule
[R¹₂R²SiO_{1/2}]₂[R²(Y')SiO_{2/2}]ₖ[R¹₂SiO_{2/2}]ₘ (I'),
R¹, R², m et k possédant la signification indiquée pour ceux-ci dans la revendication 2 et
Y' possédant la signification indiquée pour celui-ci dans la revendication 8, sont utilisés.

10. Compositions selon la revendication 8 ou 9, **caractérisées en ce qu'**en tant qu'organopolysiloxanes (A') fonctionnalisés par amino, ceux qui présentent un indice d'amine d'au plus 0,4 mmol/g sont utilisés.

11. Lessive et produit de nettoyage contenant des compositions selon l'une quelconque des revendications 1 à 7.

12. Procédé pour l'entretien et le nettoyage de fibres, en particulier de fibres textiles et de tissus textiles, avec les compositions selon l'une quelconque des revendications 1 à 7 ou avec les lessives et les produits de nettoyage selon la revendication 11 contenant ces compositions.

13. Formulations cosmétiques, en particulier produits de traitement capillaire, contenant des compositions selon l'une quelconque des revendications 1 à 7.

14. Procédé pour l'entretien et le nettoyage de cheveux avec les compositions selon l'une quelconque des revendications 1 à 7 ou avec les formulations cosmétiques selon la revendication 13 contenant ces compositions.
